# EUROPEAN PATENT SPECIFICATION

(11) **EP 0 629 132 B1**
(45) Date of publication and mention of the grant of the patent: **11.08.2004**
(21) Application number: 93906142.0
(22) Date of filing: 24.02.1993
(51) Int. Cl.: A61K 39/00, A61K 39/02, A61K 35/16, C07H 15/12, C12Q 1/00, C12Q 1/68

(54) **PURIFIED VACUOLATING TOXIN FROM HELICOBACTER PYLORI AND METHODS TO USE SAME**
GEREINIGTES VAKUOLISIERENDES TOXIN AUS HELICOBACTER PYLORI UND METHODEN ZU DESSEN VERWENDUNG
TOXINE VACUOLISANTE PURIFIEE ISSUE DE HELICOBACTER PYLORI ET METHODES D'UTILISATION DE CETTE TOXINE

(30) Priority: 26.02.1992 US 841644
(43) Date of publication of application: 21.12.1994
(73) Proprietor: VANDERBILT UNIVERSITY, Nashville, TN 37240 (US)
(72) Inventor: COVER, Timothy, L., Nashville, TN 37209 (US); BLASER, Martin, J., Nashville, TN 37205 (US)
(74) Representative: Grünecker, Kinkeldey, Stockmair & Schwanhäusser Anwaltssozietät
(86) International application number: PCT/US1993/001558
(87) International publication number: WO 1993/016723

(56) References cited:
- WO-A-93/18150
- WO-A-94/04161
- THE JOURNAL OF BIOLOGICAL CHEMISTRY, vol. 267, May 1992, pages 10570-10575, XP002019214 COVER, T.L. AND M.J. BLASER: "Purification and Characterization of the vacuolating toxin from Helicobacter pylori"
- THE JOURNAL OF BIOLOGICAL CHEMISTRY, vol. 265, 1990, pages 21940-21945, XP000605473 UMATA, T. ET AL.: "The cytotoxic action of diphtheria toxin and its degradation in intact vero cells are inhibited by bafilomycin A1, a specific inhibitor of vacuolar-type H+-ATPase"
- REVIEWS OF INFECTIOUS DISEASES, vol. 13, no. SUPPL. 08, July 1991, pages S686-S689, XP000560421 LAUNK R. D.: "PRODUCTION OF A CYTOTOXIN BY HELICOBACTE PYLORI"
- Infection and Immunity, Volume 58, No. 3, issued March 1990, COVER et al.: "Characterization of and Human Serologic Response to Proteins in Helicobacter pylori Broth Culture Supernatants with Vacuolizing Cytotoxin Activity", pages 603-610, see entire document.
- Journal of Medical Microbiology, Volume 26, issued 1988, LEUNK et al.: "Cytotoxic Activity in Broth-Culture Filtrates of Campylobacter pylori", pages 93-99, see entire document.
- Journal of Clinical Microbiology, Volume 27, No. 1, issued January 1989, FIGURA et al.: "Cytotoxin Production by Campylobacter pylori Strains Isolated from Patients with Peptic Ulcers and from Patients with Chronic Gastritic Only", pages 225-226, see entire document.

### SEQUENCE LISTING

(1) GENERAL INFORMATION:
   (i) APPLICANT: Cover, Timothy L.
      Blaser, Martin J.
   (ii) TITLE OF INVENTION: Purified Vacuolating Toxin From Helicobacter Pylori and Methods to Use Same
   (iii) NUMBER OF SEQUENCES: 22
   (iv) CORRESPONDENCE ADDRESS:
      (A) ADDRESSEE: Tilton, Fallen, Lungmus & Chestnut
      (B) STREET: 100 South Wacker Drive, Suite 960
      (C) CITY: Chicago
      (D) STATE: Illinois
      (E) COUNTRY: USA
      (F) ZIP: 60606-4002
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Fentress, Susan B.
      (B) REGISTRATION NUMBER: 31,327
      (C) REFERENCE/DOCKET NUMBER: 92104 CIP
   (ix) TELECOMMUNICATION INFORMATION:
      (A) TELEPHONE: (312)-456-8000
      (B) TELEFAX: (312)-456-7776
(2) INFORMATION FOR SEQ ID NO:1:
   (i) SEQUENCE CHARACTERISTICS:
      (A) LENGTH: 1412 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:2:
   Ala Phe Phe Thr Thr Val Ile Ile Pro Ala Ile Val
   1               5                   10
   Gly Gly Ile Ala Thr Gly Thr Ala Val Gly Thr
           15                  20
(2) INFORMATION FOR SEQ ID NO:3:
   (i) SEQUENCE CHARACTERISTICS:
      (A) LENGTH: 24 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:3:
   Ala Phe Phe Thr Thr Val Phe Gly Leu Glu Ala Ile
   1               5                   10
   Val Lys Ile Val Gly Leu Arg Tyr His Tyr Phe Thr
           15                  20
(2) INFORMATION FOR SEQ ID NO:4:
   (i) SEQUENCE CHARACTERISTICS:
      (A) LENGTH: 24 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:5:
   Ala Phe Phe Ser Thr Asn Cys Val Glu Gly Thr Ala
   1               5                   10
   Val Gly Ile Val Ile Ser Thr Gly Asp Arg Thr
           15                  20
(2) INFORMATION FOR SEQ ID NO:6:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:6:
   Val Ala Leu Leu Val Cys Leu Ile Pro Thr Thr Ile
   1               5                   10
   Gly Gly Leu Leu Ser Ala Ser Ala Val Ala Gly
           15                  20
(2) INFORMATION FOR SEQ ID NO:7:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:7:
   Glu Met Ile Met Lys Ser Ile Ile Pro Val Val Met
   1               5                   10
   Ala Gly Ile Ile Ala Ile Tyr Gly Leu Val Val
           15                  20
(2) INFORMATION FOR SEQ ID NO:8:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:8:
   Ala Phe Leu Leu Ala Val Ile Ile Gly Val Ser Ala
   1               5                   10
   Gly Val Ile Ala Ala Leu Lys Gln Asn Thr Arg
           15                  20
(2) INFORMATION FOR SEQ ID NO:9:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:9:
   Ala Phe Phe Thr Pro Phe Ile Thr Ser Ala Ile Asn
   1               5                   10
   Phe Tyr Tyr Gly Tyr Val Phe Met Gly Cys Leu
           15                  20
(2) INFORMATION FOR SEQ ID NO:10:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:10:
   Phe Phe Phe Ala Ser Leu Met Ile Leu Ser Ile Val
   1               5                   10
   Phe Val Phe Phe Leu Ile Pro Glu Thr Lys Gly
           15                  20
(2) INFORMATION FOR SEQ ID NO:11:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:11:
   Gln Phe Leu Ala Ser Val Gln Thr Met Glu Ile Val
   1               5                   10
   Pro Ser Phe Ala Val Gly Ser Ala Val Gly Leu
           15                  20
(2) INFORMATION FOR SEQ ID NO:12:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:12:
   Ala Phe Phe Ser Thr Met Cys Leu Glu Gly Thr Ala
   1               5                   10
   Gln Gly Leu Val Val Asn Thr Gly Asp Arg Thr
           15                  20
(2) INFORMATION FOR SEQ ID NO:13:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:13:
   Leu Phe Ser Gly Thr Asn Ile Ala Ala Gly Lys Ala
   1               5                   10
   Leu Gly Ile Val Ala Thr Thr Gly Val Ser Thr
           15                  20
(2) INFORMATION FOR SEQ ID NO:14:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:14:
   Glu Met Ile Met Lys Ser Ile Ile Pro Val Val Met
   1               5                   10
   Ala Gly Ile Ile Ala Ile Tyr Gly Leu Val Val
           15                  20
(2) INFORMATION FOR SEQ ID NO:15:
   (i) SEQUENCE CHARACTERISTICS:
      (A) LENGTH: 17 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:15:
   Leu Gly Gln Phe Asn Gly Asn Ser Phe Thr Ser Tyr
   1               5                   10
   Lys Asp Xaa Ala Asp
           15
(2) INFORMATION FOR SEQ ID NO:16:
   (i) SEQUENCE CHARACTERISTICS:
      (A) LENGTH: 9 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:16:
   Asn Ile Lys Asn Val Glu Ile Thr Arg
   1               5
(2) INFORMATION FOR SEQ ID NO:17:
   (i) SEQUENCE CHARACTERISTICS:
      (A) LENGTH: 21 amino acids
      (B) TYPE: amino acid
      (C) STRANDEDNESS: unknown
      (D) TOPOLOGY: unknown
   (ii) MOLECULE TYPE: protein
   (ix) FEATURE:
      (A) NAME/KEY: Modified-site
      (B) LOCATION: 3
      (D) OTHER INFORMATION: /note= "EITHER VALINE OR ISOLEUCINE CAN BE USED HERE"
   (xi) SEQUENCE DESCRIPTION: SEQ ID NO:17:
   Thr Arg Val Asp Phe Asn Ala Lys Asn Ile Leu Ile
   1               5                   10
   Asp Asn Phe Leu Glu Ile Asn Asn Arg
           15                  20
(2) INFORMATION FOR SEQ ID NO:18:
   (i) SEQUENCE CHARACTERISTICS:
      (A) LENGTH: 20 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:18:
   TTYTTYACNA CNGTNATHAT
                      20
(2) INFORMATION FOR SEQ ID NO:19:
   (i) SEQUENCE CHARACTERISTICS:
      (A) LENGTH: 23 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:19:
   GAYAAYTTYY TNGARATHAA YAA
   23
(2) INFORMATION FOR SEQ ID NO:20:
   (i) SEQUENCE CHARACTERISTICS:
      (A) LENGTH: 23 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:20:
   CTRTTRAARR ANCTYTADTT RTT
   23
(2) INFORMATION FOR SEQ ID NO:21:
   (i) SEQUENCE CHARACTERISTICS:
      (A) LENGTH: 20 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:21:
   TTTTTTACAA CCGTGATCAT
   20
(2) INFORMATION FOR SEQ ID NO:22:
   (i) SEQUENCE CHARACTERISTICS:
      (A) LENGTH: 23 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:22:
   TTATTGATTT CTAAAAAATT ATC
                          23

## Description

### Background of the Invention

This application is a continuation-in-part of U.S. Serial No. 841,644 filed February 26, 1992.

### Field of the Invention

This invention relates to a Helicobacter pylori vacuolating toxin, methods to use the toxin in diagnostic testing for the predisposition to peptic ulceration and gastric malignancy, and methods to use the toxin as a vaccine for providing immunologic protection against H. pylori infection.

### Brief Description of the Background Art

Helicobacter pylori is a curved Gram-negative bacterium that is commonly present in the human stomach; once acquired, this organism persists for years or decades (Blaser, M.J. (1990) J. Infect. Dis. 161:626-633). Multiple lines of evidence now indicate that H. pylori infection nearly universally results in chronic gastritis (Dixon, M.F. (1991) Gastroenterol. and Hepatol. 6:125-130 Although most persons with H. pylori-induced gastritis remain asymptomatic, this condition is a significant risk factor for the development of both peptic ulceration and gastric adenocarcinoma (Peterson, W. L. (1991) N. Engl. J. Med. 324:1043-1048, and Nomura, A., Stemmermann, G.N., Chyou, P.-H., Kato, I., Perez-Perez, G.I, and Blaser, M.J., N. Eng. J. Med. 1991; 325:1132-6).

The pathogenesis of H. pylori infection is not yet well understood. The production of high levels of urease by the organism (Dunn, B.E., Campbell, G.P., Perez-Perez, G.I., and Blaser, M.J. (1990) J. Biol. Chem. 265:9464-9469), is thought to be essential for the initiation and maintenance of gastric infection (Eaton, K.A., Morgan, D.R., Krakowka, S. (1989) Infect. Immun. 57:1119-1125). Another potential virulence determinant is a toxin that induces vacuolation of eukaryotic cells (Cover, T.L., Halter, S.A., Blaser, M.J. (1992) Human Pathol. 23:1004-1010. Functionally active toxin is produced in vitro by 50-60% of H. pylori isolates (Leunk, R.D., Johnson, P.T., David, B.C., Kraft, W.G., and Morgan, D.R. (1988) J. Med. Microbiol. 26:93-99 and Cover, T.L., Dooley, C.P., and Blaser, M.J. Infect. Immun.; 58:603-610 (1990)). Antibodies that neutralize toxin activity are present in sera from H. pylori-infected persons, which indicates that the vacuolating toxin activity is relevant in vivo (Leunk, R.D., Ferguson, M.A., Morgan, D.R., Low, D.E., and Simor, A.E. (1990) J. Clin. Microbiol. 28:1181-1184 and Cover, T.L., Cao., P., and Blaser, M.J. (1991) Gastroenterology 100:A570). Two studies have indicated that the prevalence of infection with toxin-producing H. pylori is higher among H. pylori-infected persons with peptic ulceration than among infected persons with gastritis alone (Figura, N., Guglielmetti, P., Rossolini, A., Barberi, A., Cusi, G., Musmanno, R., Russi, M., and Quaranta, S. (1989) J. Clin. Microbiol. 27:225-226; Goosens, H., Vlaes, L., Lambert, J.P., Glupczynski, Y., Burette, A., and Butzler, J.P. (1991) Microb. Ecol. Health Dis. 4:130).

In previous work, the inventors have identified several H. pylori proteins that are present in broth culture supernatants with vacuolating toxic activity, but absent or reduced in concentration in supernatants that lack toxic activity (Cover, T.L., Dooley, C.P., and Blaser, M.J. (1990) Infect. Immun. 58:603-610). In addition, the inventors have demonstrated that the vacuolating toxin is distinct from H. pylori urease (Cover, T.L., Puryar, W., Perez-Perez, G.I., and Blaser, M.J. (1991) Infect. Immun. 59:1264-1270). In this application the inventors describe the purification and characterization of the vacuolating toxin from H. pylori.

### Summary of the Invention

It is an object of the present invention to provide a substantially pure antigenic composition with vacuolating toxin activity. This antigenic composition can be either purified from natural material or produced recombinantly.

It is a further object of this invention to provide the gene which can be expressed to provide recombinant vacuolating toxin or fragments thereof. The partial DNA sequence of the gene is set out in sequence Id no. 1.

It is an object of the present invention to provide a purified antigenic composition that specifically binds antibodies to the toxin.

It is an object of the present invention to provide a clinical diagnostic test for the presence of infection with toxin-producing H. pylori, and thereby identify patients at risk for peptic ulcer disease or gastric malignancy.

It is an object of the invention to provide a protein vaccine which induces high levels of specific antibodies directed against H. pylori toxin, and which protects against natural H. pylori infection in humans.

It is another object of the invention to provide polyclonal or monoclonal antibodies specific for H. pylori toxin, and methods for their use in detecting the toxin, or for therapeutic purposes.

These and other embodiments are accomplished by providing the antigenic compositions, vaccines, methods, antisera or antibodies, and kits disclosed herein.

In one embodiment of the invention, a purified antigenic composition with vacuolating toxin activity (hereinafter termed CB antigen) is extracted from H. pylori broth culture supernatant, and has a molecular weight greater than 972,000 daltons (as determined by gel filtration chromatography under nondenaturing conditions), an apparent molecular weight of 87,000 ± 300 daltons when denatured (as determined by sodium dodecyl sulfate polyacrylamide gel electrophoresis under reducing conditions (SDS-PAGE) and an isoelectric point of approximately 6.1. The antigen includes the amino terminal sequence shown is Sequence Id. No. 2, the internal amino acid sequences shown in Sequence Id. Nos. 15-17 and may additionally include the amino acid sequence that is encoded by the nucleotide sequence as set out in Sequence Id. No. 1. The term CB antigen is defined as the functionally active non-denatured vacuolating toxin; in contrast, the Mr = 87,000 protein is a functionally inactive subunit of the CB antigen, which is detected only under denaturing conditions. The term CB antigen will include antigenic fragments of the holotoxin, whether derived from H. pylori or synthetically or recombinantly produced. Antigen proteins having substantial homology to CB antigen or fragments thereof may also be used in accordance with the invention. Additionally, CB antigen analogs are also contemplated.

Antiserum or monoclonal antibodies raised against CB antigen may be used to test for the presence of toxin. Test samples are contacted with such antiserum, followed by detection of antibody binding to components of the test samples. Where such binding exceeds a predetermined positive threshold level, the sample is positive for toxin.

CB antigen may be capable of inducing protective immunity against H. pylori infection when administered to humans in a nonvirulent manner. Hence, the antigen may be used in combination with a suitable adjuvant, as a vaccine against future H. pylori infection.

In one aspect of the invention, CB antigen is used in methods for the detection of anti-toxin antibodies. The purified toxin is contacted with samples of body fluids suspected of containing antitoxin antibodies. Following such contacting, known methods are used to determine the extent of antigen-antibody complex formation. When formation of the complex exceeds a predetermined positive threshold value, the test is positive for presence of anti-toxin antibodies.

Preferred techniques for detecting formation of antigen-antibody complexes include, but are not limited to, enzyme-linked immunosorbent assay (ELISA), indirect immunofluorescence assay, latex agglutination, and liposome-based assay. Alternatively, a Western blot technique may be used, in which case the bands are detected by visual inspection, and substantial appearance of dark bands may be taken as a positive indication. The extent of detection of the antigen/antibody complex which should be considered a positive signal (i.e., an indication that the test sample includes toxin-specific antibody) depends upon the detection means chosen, but may be defined generically as a value greater than the mean plus 1 interval of standard deviation from the results observed with samples from a negative control group, all other parameters (dilution of sample, time of incubation, etc.) being held constant. In some embodiments where higher specificity is desired, the mean plus two or mean plus three standard deviations may be utilized. The negative control group should consist of individuals who are known to be free of H. pylori infection.

In one aspect of the invention, kits are provided which include the antigenic compositions within the scope of the invention, and which further include means for detecting the presence of any immunoglobulin in a test sample which may become bound to antigens in said compositions.

Additionally, diagnostic tests for H. pylori infection can be developed based on primer directed amplification of nucleic acid samples of subjects. More specifically, synthetic oligonucleotides selected from the nucleotides set out in sequence Id. no 1 can be used in a polymerase chain reaction to amplify H. pylori toxin to detectable levels.

### Brief Description of the Drawings

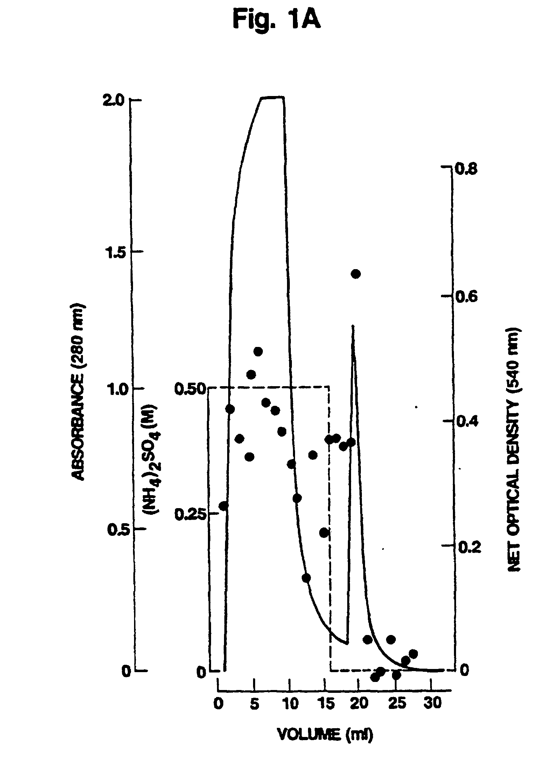

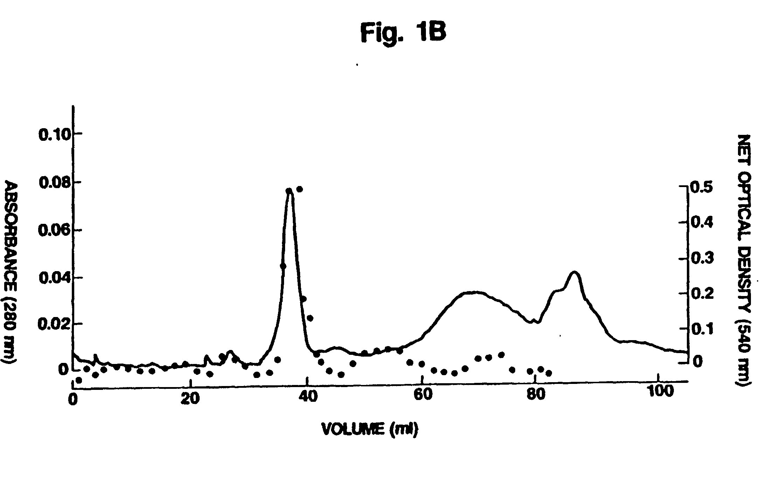

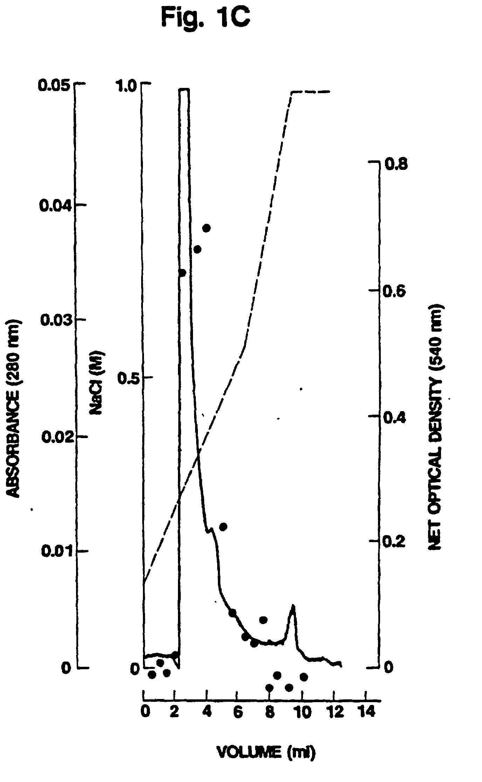

Figure 1 shows column chromatography of H. pylori vacuolating toxin. Column eluates were monitored for absorbance at 280 nanometers (solid lines), and salt concentrations are indicated by the dashed lines. The vacuolating cytotoxin activity of fractions was assayed using the neutral red assay and is expressed as net optical density (solid circles). A) PHENYLSUPEROSE (Pharmacia) chromatography of ammonium sulfate-precipitated supernatant proteins. The presence of ammonium sulfate (0.5M) in the buffer of early fractions (volume 1-15 ml) contributed to the neutral red uptake induced by these fractions (Cover, T.L., Puryear, W., Perez-Perez, G.I., Blaser, M.J. (1991) Infect. Immun. 59:1264-70). B, the eluted peak from A was applied to a SUPEROSE 12 (Pharmacia) column, and toxic activity was detected in the void volume. C, fractions with toxic activity eluted from the SUPEROSE 12 (Pharmacia) column were applied to a MONO Q (Pharmacia) column, and toxic activity was eluted by a linear gradient of NaCl.

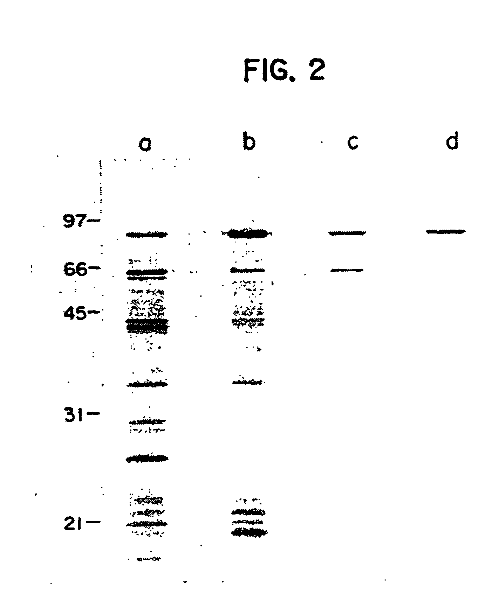

Figure 2 shows sodium dodecyl sulfate-polyacrylamide gel electrophoresis (12% acrylamide) of H. pylori toxin (CB antigen) under denaturing, reducing conditions. Lanes are: a, proteins precipitated from a broth culture supernatant of H. pylori 60190 by a 50% saturated solution of ammonium sulfate; b, toxin partially purified by hydrophobic interactive chromatography; c, toxin partially purified by gel filtration chromatography; d, purified CB antigen after anion exchange chromatography, visualized as an Mr = 87,000 band under denaturing, reducing conditions. Chromatography conditions were as described in the text. The migrations of marker proteins of known molecular weight (in kilodaltons) are shown at left.

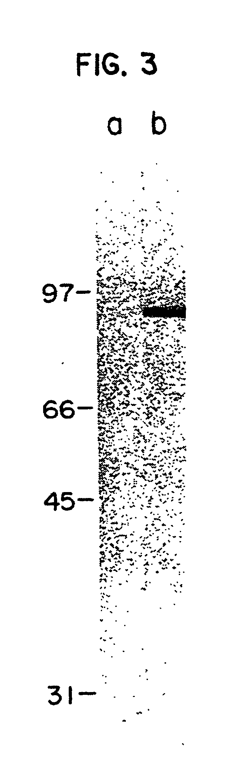

Figure 3 shows Western blot recognition of the Mr = 87,000 protein band by immune rabbit serum. Proteins precipitated from the broth culture of H. pylori 60190 by a 50% saturated solution of ammonium sulfate were electrophoresed on a 10% acrylamide gel, transferred to a nitrocellulose paper, incubated with 1:10,000 dilutions of rabbit sera, and the antigens resolved. Lane a, preimmune serum. Lane b, antiserum produced against the purified denatured Mr = 87,000 H. pylori protein subunit. The antiserum recognized only the Mr = 87,000 band.

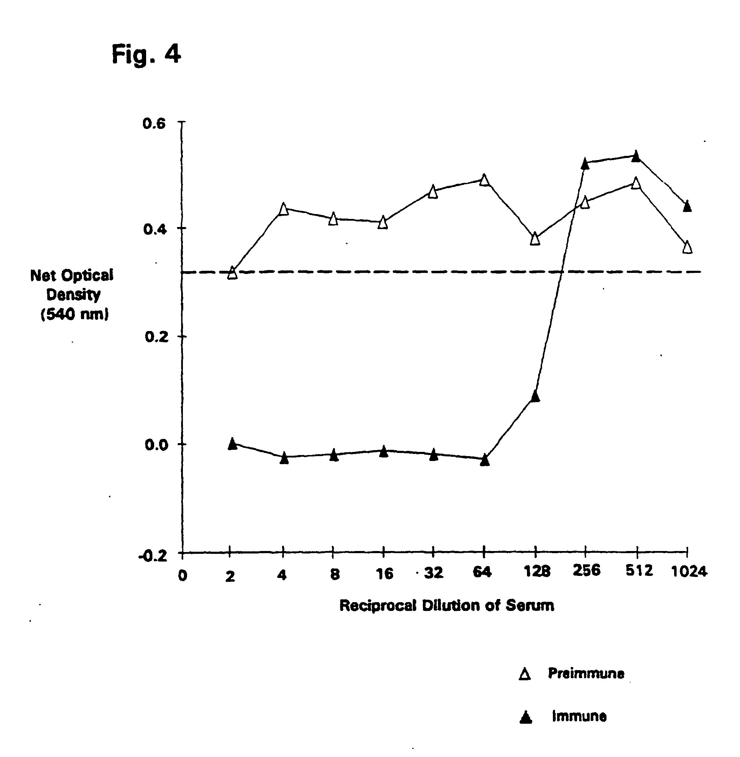

Figure 4 shows neutralization of H. pylori vacuolating toxin activity by antiserum raised against the purified denatured Mr = 87,000 protein subunit. Preimmune serum and antiserum raised against the purified Mr = 87,000 H. pylori protein subunit were tested for toxin-neutralizing activity. The neutral red uptake induced by crude concentrated broth culture supernatant from H. pylori 60190 is indicated by the dashed line. At a dilution of 1:64, the antiserum completely neutralized toxin activity, whereas the preimmune serum failed to neutralize toxin activity.

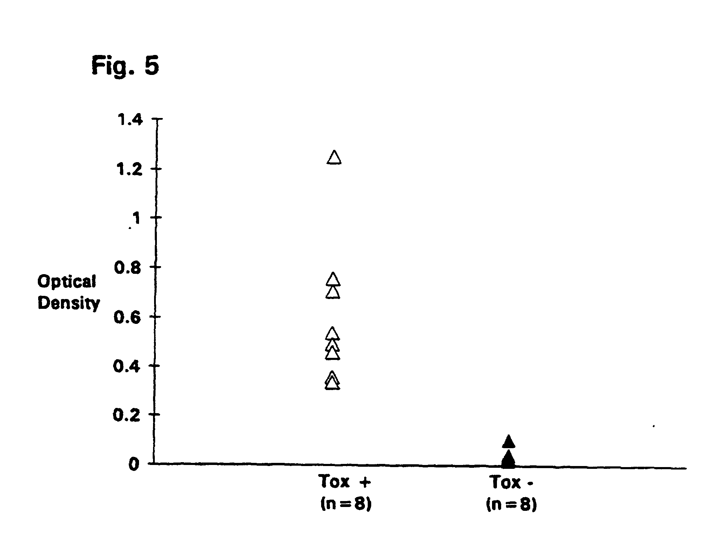

Figure 5 shows detection of the vacuolating toxin in H. pylori supernatants. Concentrated culture supernatants from 8 tox⁺ H. pylori strains and 8 tox⁻ strains were diluted 1:100 in carbonate buffer and tested in an ELISA for reactivity with antiserum to the denatured Mr = 87,000 protein subunit (1:10,000 dilution). Tox⁺ supernatants produced significantly higher optical density values than tox⁻ supernatants (0.614 ± 0.11 versus 0.046 ± 0.01 , p<0.0001).

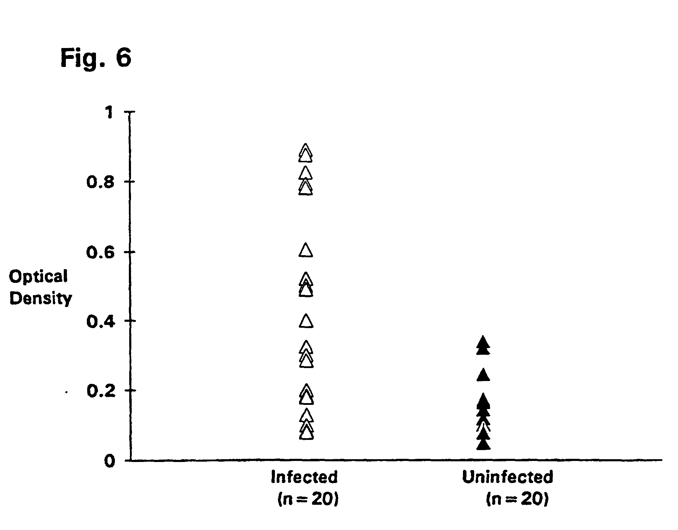

Figure 6 shows serologic recognition of the purified H. pylori toxin (CB antigen) by human sera. Sera from twenty H. pylori-infected persons and twenty uninfected persons were diluted 1:100 and tested in an ELISA for IgG reactivity with the purified CB antigen (15 ng/microtiter well). Sera from H. pylori-infected persons recognized the purified toxin significantly better than sera from uninfected persons (mean optical densities 0.424 ± 0.06 and 0.182 ± 0.02, respectively, p=0.0009).

### Detailed Description of the Invention and Best Mode

This work represents the first purification to homogeneity of the vacuolating toxin of H. pylori. The toxin was isolated from broth culture supernatant by ammonium sulfate precipitation, followed by hydrophobic interactive chromatography, gel filtration chromatography, and anion exchange chromatography. The term substantially pure means that the CB antigen is present in the antigenic composition at a concentration, relative to the other H. pylori products, higher than that in the H. pylori broth culture supernatant.

These procedures resulted in recovery of a purified, functionally active toxin, with molecular weight greater than 972,000 daltons under non-denaturing conditions (CB antigen). The purification of this protein was associated with a greater than 5000-fold increase in specific activity of the toxin. Analysis of the purified toxin (CB antigen) by SDS-PAGE under denaturing, reducing conditions demonstrated the presence of a single band migrating at 87,000 daltons. By ELISA, antiserum to the denatured Mr = 87,000 protein subunit reacted with tox⁺ H. pylori supernatants to a significantly greater extent than with the tox⁻ H. pylori supernatants. In addition, antiserum to the denatured Mr = 87,000 protein subunit neutralized the vacuolating toxic activity of H. pylori 60190, as well as the toxins produced by other H. pylori strains. Altogether, these data support the role of the CB antigen in vacuolating toxin activity, and indicate that the toxins produced by various H. pylori strains are antigenically related.

Western blot analysis demonstrated that the denatured Mr = 87,000 protein subunit appears related to a band identified in our previous analysis of tox⁺ H. pylori culture supernatants (originally reported as Mr = 82,000) (Cover, T.L., Dooley, C.P., and Blaser, M.J. (1990) Infect. Immun. 58:603-610). Our previous study of tox⁺ H. pylori supernatants also identified an Mr = 128,000 protein, which was recognized by sera from patients with peptic ulceration more frequently than by H. pylori-infected persons without ulcer disease (Cover, T.L., Dooley, C.P., and Blaser, M.J. (1990) Infect. Immun. 58:603-610; Crabtree, J.E., Taylor, J.D., Wyatt, J.I., Heatley, R.V., Shallcross, T.M., Tompkins, D.S., and Rathbone, B.J. (1991) Lancet 338:332-335). The current study indicates that the Mr = 128,000 protein is not required for expression of vacuolating toxin activity, and is not immunologically cross-reactive with the Mr = 87,000 protein subunit.

Any sample suspected of containing antibodies may be tested in accordance with the methods set forth herein. Preferably, the samples to be tested are bodily fluids such as blood, serum, urine, tears, saliva and the like. In addition to human samples, samples may be taken from mammals such as non-human primates, horses, swine, etc. Due to the sensitivity of the test described, it is possible to dilute the sample prior to testing. Dilution may proceed by addition of any fluid compatible with each of the sample, the antibodies to be tested, and the antigenic composition. Serum, when used as the sample, may, for example, be diluted with one or more fluids selected from the group consisting of phosphate-buffered saline, pH 7.0-7.4 (hereinafter, "PBS"), PBS-containing Tween 20 (hereinafter,"PBS T"); PBS T with thimerosal (hereinafter, "PBS" TT), PBS TT with gelatin (hereinafter, "PBS TTG"), and PBS TTG with bovine gamma globulin (hereinafter, "PBS TTGG"). Dilutions, when testing for IgG antibody, may be as high as a ratio from about 1:100 to about 1:1000. Although samples also may be tested for IgA and IgM antibodies, IgG tests are preferred.

Preferred diluents and dilution ratios may vary according to the sample being tested. Urine, for instance, is already relatively dilute and may not need to be diluted further. However, it may not be necessary to concentrate urine as is often necessary with other assays. Prior to testing, the pH of urine is preferably adjusted to between about 7.0 and 7.4, the preferred pH for antibody function.

While dilution of sample is not required, it is believed that dilution reduces the possibility that significant antigen/antibody complexes will be formed in the absence of H. pylori specific antibodies. The extent of dilution should be taken into account in adjusting the threshold level of antigen/antibody complex which should be considered a positive signal.

While the present disclosure provides an easy method for obtaining the purified toxin (CB antigen) from the deposited H. pylori strain, it is emphasized that this antigen is common to a number of H. pylori strains. While the deposited strain and the description of the present specification provide an easy manner of isolating this antigen, it is emphasized that the present invention broadly encompasses use of the antigen regardless of the source or method whereby it is derived, such as for example by recombinant production.

Before contacting a test sample with antigenic compounds in accordance with the invention it is preferred (but not necessary) that the antigenic composition be immobilized using conventional techniques. In one alternative embodiment, liposome-based assays may be used as described in more detail below. For conventional immobilization, polystyrene plates, for example, may be incubated with antigenic suspensions made in accordance with the invention. Alternatively, for example, antigens isolated as protein bands on electrophoretic gel may be transferred to a nitrocellulose sheet by known methods. See Towbin et al., Proc. Nat'l. Acad. Sci., 76:4350-54 (1979); Burnette et al., Biochem., 112:95-203 (1981). Numerous other techniques are known in the art for binding antigens to substantially inert substrates.

Bound antigens in accordance with the invention are preferably contacted with a dilute fluid which includes the sample to be tested for presence of antibody to H. pylori. The antigen and sample are preferably incubated for at least 5 to 15 minutes. Less time is needed when incubation proceeds at or near human body temperature, about 37°C. Incubation at other temperatures, for instance 4°C, is also proper, but generally requires additionally incubation time. Preferred incubation time at 37°C is from about 5 minutes to about 90 minutes. Rapid assays can also be performed at room temperature. The bound antigens should then be rinsed to remove any unbound antibodies, i.e., those which are not specific for the antigens. Preferably, rinsing proceeds with a buffer solution such as PBS T, PBS TT or Tris/Tween/Sodium chloride/azide. Multiple rinsing are preferred.

During incubation, H. pylori specific antibodies bind to the immobilized antigens to create antigen/antibody complexes. All unbound antibodies are substantially removed during the rinsing procedure. Due to the high specificity of the antigens of the invention, antibodies which are not specific for H. pylori are substantially removed by the rinsing. Naturally, if the tested sample did not contain H. pylori specific antibodies, the immobilized antigens would be substantially free of human antibody, and subsequent testing for antigen/antibody complexes should not indicate a substantial presence of such complexes. On the other hand, if the tested sample were rich in H. pylori specific antibodies, these antibodies should have bound to the immobilized antigens to form a large quantity of antigen/antibody complex for subsequent detection.

Detection of antigen/antibody complex may be achieved by a wide variety of known methods. Preferred methods include but are not limited to enzyme-linked immunosorbent assay, latex agglutination, Western blot technique or indirect immunofluorescence assay.

Typically, the H. pylori specific antibodies complexed with immobilized antigen are detected by contact with labeled or otherwise detectable second antibodies specific for the immunoglobulin being tested for. If the test sample is human sera, for example, the detectable second antibody is specific for human immunoglobulin. The labeled second antibodies may be specific for any human antibody, preferably of the IgG or IgA type, most preferably IgG. When acute sero-conversion is suspected, an IgM test using a labeled second antibody specific for IgM may be appropriate. The second antibodies are preferably incubated with the immobilized antigens for about 5 minutes to about two hours, preferably 30 minutes to 60 minutes at a temperature of about 20°C to about 37°C. Then, the antigens are washed with a buffer solution (preferably multiple times) in order to remove all unbound labeled antibody. The washing will remove substantially all labeled antibody except that which has bound to immunoglobulin present on the antigens. Of course, substantially the only human immunoglobulin present at this point should be H. pylori specific antibody. Hence, the presence of H. pylori specific antibody may be indirectly measured by determining the presence or absence of the labeled second antibody.

There are many known techniques for detecting the label, which vary with the type of label used. For instance, fluorescein-labeled antibody may be detected by scanning for emitted light at the characteristic wavelength for fluorescein. Alternatively, an enzyme label is detected by incubation with appropriate substrates and detection of an enzyme activity, preferably activity resulting in a color change. Such activity can be determined by visual inspection or can be read automatically by a spectrophotometer set at the appropriate wavelength.

Alternatively, the enzyme label may be horseradish peroxidase and the substrate may be H₂O₂ and 2,2'-azinobis(3-ethylbenzothiazoline-6-sulfonic acid) which produces in the presence of the enzyme, a compound detectable by a spectrophotometer set at 414 nm.

In Western blotting, the positive signal may be detected when an enzyme is conjugated to the second antibody. Incubation with appropriate substrate enzymatically produces a color product in the immediate vicinity of the antigenic band resolved by this process. The presence of a reactive band may be detected by visual inspection. In an indirect immunofluorescence assay, fluorescein-labeled second antibodies may be detected by flurorescence-activated detectors, or by visual inspection.

A liposome-based assay may involve the presence of fluorescein, an enzyme or a substrate inside a liposome onto whose surface H. pylori antigens are expressed. These liposomes are incubated with a diluted body fluid sample to be tested, and are thoroughly washed. Any liposome with immunoglobulins on their surface forming an antigen/antibody complex may be recognized by attaching a second antibody, specific to the immunoglobulin being tested for, onto the inside walls of a polystyrene tube containing the liposomes. Liposomes having antibody bound to their surfaces will become immobilized on the tube walls, and non-immobilized liposomes will be washed away. The liposomes can by lysed with, for instance, detergent, or complement, and the enzyme or substrate that was in the interior is now free to react with the complementary substrate (or enzyme) in the solution in the tube. Enzymatic activity, preferably a color change reaction could be detected by visual inspection or spectrophotometric color determination. Enzymatic activity beyond the predetermined positive threshold indicates the presence of H. pylori specific antibodies.

The sensitivity and specificity of the antibody detection in accordance with the present invention have been determined using serum obtained from persons from defined populations. By ELISA, IgG antibodies to the purified toxin (CB antigen) have been identified in sera from H. pylori-infected persons. The ELISA optical density values produced by sera from approximately 50% of H. pylori-infected persons exceeded the range produced by sera from uninfected persons. This suggests that approximately 50% of H. pylori-infected persons are infected with strains of H. pylori that produce the toxin. Similarly, approximately 50% of H. pylori strains produce the toxin in vitro. (Leunk, R.D., Johnson, P.T., David, B.C., Kraft, W.G., and Morgan, D.R. (1988) J. Med. Microbiol. 26:93-99; Cover, T.L., Dooley, C.P., and Blaser, M.J. (1990) Infect. Immun. 58:603-610).

In this application results are expressed as the mean ± SEM. Optical density values were compared using the two-tailed Student's t test for independent variables.

Additionally, detection of nucleic acid in specimens comprising body fluids or tissues can be difficult because of the small quantity of nucleic acid present or because of the presence in the specimen of other interfering materials, including DNA or RNA from a different source. These limitations may be overcome by employing an analytic method referred to as the polymerase chain reaction (PCR) technique. By this technique, selective enrichment of a specific DNA sequence can be achieved by exponential amplification of the target sequence. Mullis, et al., Met. Enzymol., 155, 335 (1987). A method to detect H. pylori toxin is provided by using primer directed amplification of oligonucleotides selected from the DNA sequence set out in sequence Id. number 1, to amplify a sample of H. pylori toxin producing nucleic acids to a detectable level.

To facilitate PCR amplification, pairs of oligonucleotide primers may be employed as described in United States patent 4,683,202 (hereby incorporated by reference). The primers are designated to hybridize with sequences that flank the target DNA. Following in vitro amplification, the amplified target sequence is detected by a hybridizing probe. For example, this analytical procedure has been used for the direct detection of HIV-1 as described by Ou, et al., Science, 238, 295-97 (1988). The amplification cycles are facilitated by using a polymerase which is thermally stable in incubations up to 95 degrees centigrade, as described by Saiki, et al., Science, 239, 487-91 (1988).

Certain embodiments of the present invention used synthetic oligonucleotide sequences as primers. These sequences can be prepared by well known chemical procedures, and commercially available DNA synthesizers can also be used. For example, the required sequence can be prepared by the synthesis method described by Beaucage, et al., Tetrahedron letters, 22: 1859-62 (1981). Another method for the synthesis of oligonucleotide on the solid support is described in U.S. patent no. 4,458,066. Automated DNA synthesis apparatus can be used such as the DNA synthesizer sold by Applied Biosystems.

More specifically, the oligonucleotide sequences of the probe sequences are represented by the standard letter abbreviations in which the nucleotide are designate as follows: A for adenosine, T for thymidine, G for guanosine, and C for cytosine and N for unknown. These strands are represented in a standard 5' prime to 3' prime orientation. Abbreviations used in the degenerate primers are shown in Table 3.

### EXAMPLE 1

### Purification of Toxin

H. pylori 60190 (ATCC 49503), a previously described toxin-producing strain, was used as the source for toxin purification. H. pylori 60190 was cultured for 48 hours at 37°C in Brucella broth containing 0.5% charcoal (untreated, granular 8-20 mesh, Sigma) in an ambient atmosphere containing 50 CO₂ (Cover, T.L., Puryear, W., Perez-Perez, G.I., and Blaser, M.J. (1991) Infect. Immun. 59:1264-1270). The culture was centrifuged at 10,000g for 20 minutes, and proteins present in the supernatant were precipitated with a 50% saturated solution of ammonium sulfate. After centrifugation at 10,000g for 15 minutes, the pellet was resuspended in 60mM Tris-HCl (pH 7.7).

Hydrophobic interactive chromatography was performed on a PHENYLSUPEROSE HR 5/5 column (Pharmacia LKB Biotechnology Inc., Piscataway, NJ) with buffer containing 60mM Tris-HCl and 0.5M ammonium sulfate (pH 7.7), and proteins were eluted with 60mM Tris HCl (pH 7.7). Size exclusion chromatography was performed on a SUPEROSE 12 HR 16/50 column (Pharmacia) with buffer containing 60mM Tris-HCl and 0.1M NaCl (pH 7.7) at a flow rate of 0.12 ml/minute. Anion exchange chromatography was performed on a MONO-Q HR 5/5 column (Pharmacia) in 20mM Tris (pH 7.7). Proteins were eluted with 20mM Tris containing a linear gradient of 0.3M NaCl to 0.6M NaCl over 10 milliliter. Column eluates were monitored for UV absorbance at 280 nanometers.

HeLa cells were cultured in Eagle's modified minimal essential medium containing 10% fetal bovine serum (MEM-FBS) in 96-well plates, as previously described (Cover, T.L., Dooley, C.P., and Blaser, M.J. (1990) Infect. Immun. 58:603-610). Toxin preparations were serially diluted in MEM-FBS, and 10 microliter aliquots were incubated with adherent cells and 90 microliters of medium in 96-well plates for 18 hours at 37°C. Cell vacuolation was then quantitated spectrophotometrically using a neutral red uptake assay, as previously described (Cover, T.L., Puryear, W., Perez-Perez, G.I., and Blaser, M.J. (1991) Infect. Immun. 59:1264-1270). The titer of toxic activity in a sample was defined as the maximum dilution of the sample that produced an optical density value greater than or equal to three SD above that produced by medium alone. The specific activity of a sample was defined as the ratio of the reciprocal toxin titer to the protein concentration (in mg/ml). For determination of specific activity, MEM-FBS was supplemented with ammonium chloride (10 mM), a concentration previously shown to potentiate toxic activity (Cover, T.L., Puryear, W., Perez-Perez, G.I., and Blaser, M.J. (1991) Infect. Immun. 59:1264-1270), and which approximates the concentration of ammonium ion in the gastric juice of H. pylori-infected humans (Marshall, B.J., and Langton, S.R. (1986) Lancet i:965-966).

Protein concentrations were measured using either QUANTIGOLD reagent (Diversified Biotech, Newton Centre, MA) or the BCA protein assay reagent kit (Pierce, Rockford, Illinois), depending on the concentration of samples, and albumin was used as a standard. SDS-PAGE was performed in a modified Laemmli gel system as described by Ames (Ames, G.F.-L (1974) J. Biol. Chem 249:634-644), and proteins were resolved in gels using the silver stain of Oakley et al. (Oakley, B.R., Kirch, D.R., and Morris, N.R. (1980) Anal. Biochem. 105:361-363). Molecular weight standards included rabbit muscle phosphorylase b (97,400), bovine serum albumin (66,200), hen egg white ovalbumin (45,000), bovine carbonic anhydrase (31,000), and soybean trypsin inhibitor (21,500) (Biorad, Richmond, CA).

The purification of the vacuolating toxin of H. pylori involved ammonium sulfate precipitation of proteins present in broth culture supernatant, followed by sequential hydrophobic interactive, gel filtration, and anion exchange chromatography, as described above. SDS-PAGE under denaturing conditions, and silver staining indicated purification to homogeneity of an Mr = 87,000 ± 300 protein subunit (Figure 2). As summarized in Table 1, analysis of the specific activities at each stage in the purification process indicated that the toxin (CB antigen) was purified more than 5000-fold from the unconcentrated broth culture supernatant and 25-fold from the ammonium sulfate precipitate. Thus, a substantially pure preparation was obtained in which the toxin was present at a concentration, relative to other H. pylori products, higher than that in H. pylori broth culture supernatant. The recovery of the purified toxin was 8 micrograms per liter of culture supernatant, which represented less than 5% of the toxic activity present in the original unconcentrated supernatant.

In addition to the above discussed purification method, substantially pure toxin can be produced by substituting a SUPEROSE 6 (Pharmacia) column in place of a SUPEROSE 12 (Pharmacia) column. Similarly, other modifications in the purification method, can be employed by one skilled in the art. For example, additives to prevent protein degeneration may be included, such as PMSF, DTT, EDTA AND 10% glycerol.

**Table 1.**

| Purification of vacuolating cytotoxin activity from H. pylori strain 60190 | | |
|---|---|---|
| Purification step | Specific activity | Purification (-fold) |
| Broth culture supernatant | 4.5 ± 1.5 | 1 |
| Ammonium sulfate precipitate | 950 ± 530 | 211 |
| Phenylsuperose chromatography | 2000 ± 310 | 444 |
| SUPEROSE 12 chromatography | 16,000 ± 5900 | 3556 |
| MONO Q chromatography | 24,000 ± 5600 | 5333 |
| ¹The results of three purification are shown (mean ± SEM). Chromatography conditions were as specified in the text. Specific activity was defined as the ratio of the reciprocal titer of toxic activity to the protein concentration (in mg/ml). | | |

### EXAMPLE 2

### Characterization of the CB Protein.

After partial purification by hydrophobic interactive and gel filtration chromatography, the toxin preparation was electrophoresed under denaturing conditions on a 7% acrylamide gel. The Mr = 87,000 band was excised and eluted from the gel and 0.7M ammonium bicarbonate was added. The solution was then applied to a PHENYLSUPEROSE HR 5/5 column (Pharmacia), and eluted with distilled water. Amino-terminal amino acid sequencing was performed as described previously (Pei, Z., Ellison, R.T., III, Lewis, R.V., and Blaser, M.J. (1988) J. Biol. Chem. 263:6416-6420), and the National Biomedical Research Foundation and Swiss-Prot data bases were searched for potential homologies with known proteins. Amino acid composition analysis was performed as described by Jones (Jones, B.N. (1981) J. Liq. Chromatogr. 4:565:586).

The amino acid composition of the purified, denatured Mr = 87,000 protein subunit is as follows (in mole %): Asx 14.8, Glx 9.6, Ser 9.3, His 1.5, Gly 13.0, Thr 6.7, Arg 3.5, Ala 8.1, Tyr 3.8, Met 2.3, Val 6.7, Phe 4.6, Ile 6.7, Leu 9.3 (Lys, Trp, Pro, and Cys not determined). Based on two determinations, the sequence of the 23 N-terminal amino acids is as shown in Table 2 (Sequence Id. No. 2). The N-terminal sequence is rich in hydrophobic amino acids, is uncharged, and has a predicted isoelectric point of 5.83. Garnier-Robson structural predictions indicate that this part of the sequence is associated with a 100% extended conformation.

A comparison between the N-terminal sequence of the Mr = 87,000 protein subunit and other known proteins indicated no strong homology. However, there was partial homology between the N-terminus of the Mr = 87,000 protein subunit and internal sequences of numerous other known proteins, many of which were involved in transport processes (Table 2) (Salkoff, L., Butler, A., Scavarda, N., and Wei, A. (1987) Nucleic Acids Res. 15:8569-72; Rogart, R.B., Cribbs, L.L., Muglia, L.K., Kephart, D.D., and Kaiser, M.W. (1989) Proc. Natl. Acad. Sci. USA 86:8170-74; Takeyasu, K., Tamkun, M.M., Renaud, KJ., and Fambrough, D.M. (1988) J. Biol. Chem. 263:4347-54; Hesse, J.E., Wieczorek, L., Altendorf, K., Reicin, A.S., Dorus, E., and Epstein, W. (1984) Proc. Natl. Acad. Sci. USA 81:4746-50; Mandel, M., Moriyama, Y., Hulmes, J.D., Pan, Y-C. E. Nelson, H., and Nelson, N. (1988) Proc. Natl. Acad. Sci. USA 85:5521-24; Hiles, I.D., Gallagher, M.P., Jamieson, D.J., and Higgins, C.F. (1987) J. Mol. Biol. 195:125-42; and Szkutnicka, K., Tschopp, J.F., Andrews, L., and Crillo, V.P. (1989) J. Bacteriol 171:4486-93; Hawkins, A.R., Lamb, H.K., Smith, M., Keyte, J.W., and Roberts, C.F. (1988) Mol. Gen. Genet. 214:224-231; Goldrick, D., Yu, G.-Q., Jiang, S.Q., and Hong, J.-S. (1988) J. Bacteriol 170:3421-3426). Based on hydropathy plot analyses, the sequences homologous to the H. pylori Mr = 87,000 protein subunit were frequently hydrophobic, membrane-spanning segments. In addition to the proteins listed in Table 2, there was partial homology with the calcium channel release protein from pig (Harbitz, I., Chowdhary, B., Thomsen, P.D., Davies, W., Kaufmann, W., Kran, S., Gustavsson, I., Christensen, K., and Hauge, J.G. (1990) Genomics 8:243-248), the kainate gated ion channel precursor from rat (Hollmann, M., O'Shea-Greenfield, A, Rogers, S.W., and Heinemann, S. (1989) Nature 342:643-8), general amino aid permease from Saccharomyces cerevisiae (Jaunizux, J.-C., and Grenson, M. (1990) Eur. J. Biochem. 190:39-44), arginine permease from S. cerevisiae (Hoffmann, W. (1985) J. Biol, Chem. 260:11831-7), lactose permease from E. coli ( D.E., Groneborn, B., and Muller-Hill, B. (1980) Nature 283:541-545), and the mannose permease EII-P MAN segment from E. coli (Erni, B., Zanolari, B., and Kocher, H.P. (1987) J. Biol. Chem 262:5238-47). The partial homology between the N-terminus of the Mr = 87,000 protein subunit and different regions of multiple families of ion channel and transport proteins suggests that this relationship may be significant.

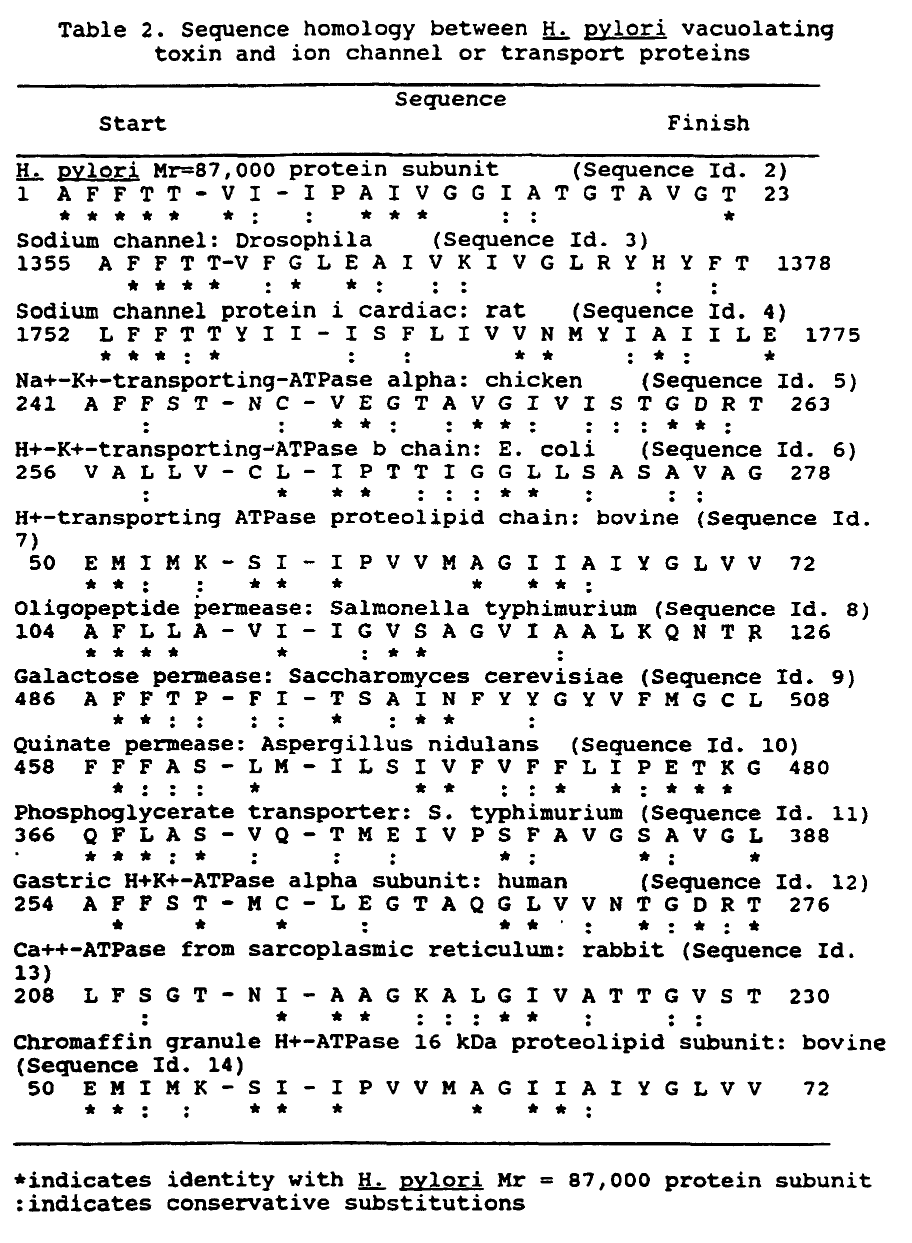

Determination of the molecular mass of the non-denatured toxin (CB antigen) was performed on a SUPEROSE 6 HR 10/30 column (Pharmacia) with buffer containing 60mM Tris-HCl and 0.1M NaCl (pH 7.7). Standards (Sigma, St. Louis, MO) included salmon sperm DNA (void volume), blue dextran (2,000,000), bovine thyroglobulin (669,000), horse spleen apoferritin (443,000), beta-amylase from sweet potato (200,000), bovine serum albumin (66,000), and carbonic anhydrase from bovine erythrocytes (29,000). The toxin preparation used in this analysis was partially purified by hydrophobic interactive and gel filtration chromatography, and then applied to the SUPEROSE (Pharmacia) 6 HR 10/30 column. Vacuolating toxin activity, as detected in cell culture, as well as the Mr = 87,000 band detected by SDS-PAGE, were present in several fractions, each with an Mr greater than 972,000, suggesting aggregation. To determine whether aggregation resulted from processes used in the purification, unconcentrated broth culture supernatant from H. pylori 60190 was passaged through the same column, and fractions were analyzed in an ELISA for reactivity with antiserum to the Mr = 87,000 protein subunit. Multiple fractions containing proteins with calculated molecular weights greater than 100,000 were recognized by the antiserum, an indication that aggregation of the Mr = 87,000 protein subunit also occurred in unprocessed supernatant.

The pI of the purified toxin was determined by isoelectric focusing. Isoelectric focusing was performed on a Resolve Alpha horizontal electrophoresis unit (Isolab, Inc., Akron, OH) using a 5% acrylamide gel (LKB, Bromma, Sweden) containing 5M urea and 2.5% ampholytes (pH range 3.5-9.5). Standards (Sigma) were trypsin inhibitor (4.6), beta-lactoglobulin A (5.13), bovine carbonic anhydrase II (B) (5.9), and human carbonic anhydrase B (6.6). The purified denatured H. pylori Mr = 87,000 protein subunit and isoelectric focusing standards were transferred to nitrocellulose paper by electroblotting for one hour. The standards were resolved by staining with Coomasie blue, and the H. pylori protein was resolved by immunoblotting with specific antiserum, using the methods described below. The non-denatured toxin failed to migrate in a 1% agarose gel (Isolab, Akron, Ohio), presumably due to its large size, Therefore, the Mr = 87,000 protein subunit was eluted from an SDS-PAGE gel fragment, and focusing in a 5% acrylamide gel containing 5M urea indicated a pI of approximately 6.1.

### EXAMPLE 3

### PCR Amplification of a Toxin-Encoding DNA Sequence and Cloning of Toxin Gene

In order to determine internal amino acid sequences, the CB antigen from H. pylori 60190 was purified by column chromatography as described by Cover et al. in Purification and Characterization of the Vacuolating Toxin from Helicobacter pylori, J. Biol. Chem. 267: 10570-575 (1992). See Example 1 for a description of the chromatography.

The purified 87 kDa band was immobilized on PVDF paper, and excised. Amido-black-stained excised protein bands were washed once with Milli-Q water and destained with 0.5 ml of 200 µM NaOH/20% acetonitrile for 1 minute, followed by one wash with Milli-Q water. The remaining nonspecific protein binding sites were blocked with 0.5 ml of 0.2% PVP-40/methanol (w/v) at room temperature for 30 minutes followed by addition of 0.5 ml of Milli-Q water. Samples were washed 6-10 times with 1 ml Milli-Q water to remove excess PVP-40, cut into approximately 1 x 1-mm squares, and returned to the same Eppendorf tube. Fifty microliters of 1% RTX-100/10% acetonitrile/100 mM Tris-HCL, pH 8.0, was added to the strips, followed by 5 µl of Arg-C protease. The best results were obtained with digestion buffer volume less than 50 µl. Digestion was carried out at 37°C for 24 hours. In experiments utilizing >0.001% SDS in the digestion buffer, an extraction with heptane/isoamyl alcohol (4:1, v/v) was performed prior to HPLC analysis. Frank, R.W., and Bosserhoff, A. (1988) in Methods in Protein Sequence Analysis (Wittmann-Liebold, B., Ed.), pp. 273-279, Springer Verlag, Berlin Heidelberg. Following digestion, samples were sonicated for 5 minutes and then centrifuged at 1700 rpm for 5 minutes, and the supernatant was transferred to an HPLC injection vial (Hewlett-Packard). Consecutive washes with 50 µl of digestion buffer (1X) and 50 µl 1.1% TFA (2X) were performed with sonication and centrifugation as described above. All supernatants were pooled for a total of 200 µl. If samples were not immediately analyzed by HPLC, the digestion was stopped by addition of 2 µl of 1% DFP/ethanol (v/v) to the pooled supernatants and the vial stored at -20°C.

Peptide isolation was performed on a 1090M HPLC (Hewlett-Packard, Avondale, PA) equipped with a binary solvent delivery system, a diode array detector, a variable-volume injector, and an autosampler. The effluent from the flowcell was directly attached to a fraction collector using capillary tubing that had a dead volume of 9 µl. Data were collected using Hewlett-Packard 79995A Chem-Station software. Peptides were injected (200 µl) and separated on a Vydac C₁₈ column (2.1 x 250 mm) with a flow rate of 150 µl/minutes at room temperature. Chromatographic conditions were as described by Stone et al. Stone, K.L., LoPresti, M.B., Williams, N.D., Crawford, J.W., DeAngelis, R., and Willaims, K.R. (1989) in Techniques in Protein Chemistry (Hugli, T.E., Ed.), pp. 377-390, Academic Press, New York. Briefly, the gradient was 1.6-29.6% B (0-63 minutes), 29.6-60%B (63-95 minutes), 60-80%B (95-105 minutes), and the buffers were A=0.1% TFA/Milli-Q water and buffer B=0.08% TFA/acetonitrile. The column was then washed at 80% B for 12 minutes at 150 µl/minute and reequilibrated at 1.6% B for 50 minutes at 300 µl/minute. Peptide elution was monitored at 220 nanometers. Fractions were collected every 0.5 minutes (75 µl/fraction) and stored at -20°C until sequence analysis was performed. Microseqencing of internal peptides was then performed as described in Fernandez (Anal. Biochem. 1992; 201:255-264).

Microsequencing of three peptides yielded the following amino acid sequences (parentheses indicate ambiguous residues);

Using this information degenerate oligonucleotide primers were synthesized corresponding to amino acid residue numbers 2-8 of Sequence Id. no. 2 and amino acid residues numbers 13-20 of the internal peptide previously designated Sequence Id. no. 17.

**TABLE 3**

| | |
|---|---|
| R=A,G | H=A,T,C, NOT G |
| Y=C,C | B=G,T,C, NOT A |
| M=A,C | V=G,A,C, NOT T |
| K=G,T | D=G,A,T,NOT C |
| S=G,C | N=G,A,T,C |

Symbols used in the degenerate primers are shown in table 3.

The degenerate oligonucleotide primers were synthesized using standard phosphoramididite chemistry on a DNA synthesizer. These degenerate oligonucleotides (Sequence Id Nos. 18 and 20) were used as primers in a polymerase chain reaction designed to amplify the intervening sequences of the toxin gene. H. pylori 60190 cells were harvested from blood agar plates in distilled water, boiled for five cycles at 100°C, and centrifuged to remove debris; the supernatant was used as the DNA template for PCR.

PCR was performed using a Perkin Elmer DNA thermal cycler according to the manufactures instructions. PCR was performed for five cycles using temperatures of 94°C for 1.5 minutes, 37°C for two minutes, and 72°C for two minutes, followed by forty-five cycles using temperatures of 94°C for 1.5 minutes, 41°C for two minutes, and 72°C for two minutes. Analysis of the amplified DNA by agarose gel electrophoresis revealed a dominant band migrating at 0.5 kb.

The 0.5 kb amplified DNA fragment was cut from the agarose gel, purified, and cloned into NOVABLUE (Novagen) cells using a pT7blue T-vector kit (Novagen). Plasmid DNA was purified from the transformant, and digestion with PstI and BamHI confirmed the presence of the 0.5 kb insert. Dideoxynucleotide sequencing of the insert using lambda ZAP vector primers was performed using standard methodology. Sanger et al., Proc. Nat'l. Acad. Sci. USA 71:1342-46 (1977), and Maniatis, et al., Molecular Cloning: A Laboratory Manual Cold Spring Harbor, NY, 1989. The nucleic acid sequence of the amplification product is set out in sequence Id. no.1 ( in the 5' to 3' direction bases 24-495). Portions of this sequence correctly encoded the amino acid sequences Id. Nos. 2, 15, and 17, indicating that a portion of the toxin gene had been successfully cloned. Specifically, sequence Id. No. 15 was encoded by bases 400-450 of sequence Id. No. 1.

To clone the entire toxin gene, chromosomal DNA from H. pylori 60190 was sheared by sonication and the resulting fragments electrophoresed on a 0.7% agarose gel. Fragments in the 2-10 kb size range were excised, treated with T4 DNA polymerase to produce blunt ends, and ligated to phosphorylated EcoRI octamer linkers (New England Biolabs). The DNA was digested EcoRI and ligated to the EcoRI arms of lambdaZAP vector (Stratagene). The ligation mixtures were added to Gigapack II packaging mix (Stratagene) and titered on XL1-blue cells. The cloned 0.5 kb fragment of the toxin gene described above was radiolabelled by primer extension using random hexamers, and used to screen the genomic library. From eight reactive clones, the recombinant phagemids were excised and transformed into XL1-blue cells. Plasmid DNA from these clones revealed DNA inserts of 1.2, 1.1, or 2.2 kb. Three representative clones containing these insert sized were used in further studies (p1A1, p3A1, and p3C), respectively. Restriction endonuclease treatment of these recombinant plasmids revealed no overlap between p1A1 and p3A1.

Partial sequences of p1A1 and p3A1 were determined using both forward and reverse vector primers. DNA sequence corresponding to the N-terminal amino acid sequence of the toxin was identified in p3A1, and sequences corresponding to internal amino acid sequence set out in sequence Id. nos. 15 and 17 were identified in p1A1. The 1.1 kb insert in p3A1 contained sequence corresponding to bases 1-177 of sequence Id. No. 1, as well as approximately 0.9 kb of an upstream sequence. The 1.2 kb insert in p1A1 contained sequence corresponding to bases 178-1412 of the sequence set out in sequence Id. no. 1.

### Expression of Recombinant Toxin in E. Coli

Clone 3A1 contained 177 bases of the toxin gene (including the N-terminal amino sequence), a leader sequence, probable promoter, and approximately 0.8 Kb of upstream sequence. Clone 1A1 contains 1.2 kb of internal toxin gene (sequences 177-1412), and flanks the EcoR1 site which is at the downstream end of 3A1. One approach to express the toxin recombinantly is to PCR amplify the 1412 basepair fragment of the toxin gene, using as primers bases 1-20 and bases 1392-1412 of sequence Id no. 1. This sequence can then be subcloned into pBluescript in E. coli XL1Blue, and the recombinant toxin can be expressed. Alternatively, the DNA inserts in 3A1 and 1A1 can be excised from the pBlusescript plasmid and gel purified. Insert 3A1 can be cut at an internal restriction site, and the fragments of 3A1 gel-purified. The fragment of 3A1 containing the desired portion of the toxin gene (promoter, leader sequence, and bases 1-177) can be ligated to insert 1A1 and subcloned into pBluescript. In both of these cases, production of recombinant toxin by pBluescript may be inducible by IPTG. If recombinant toxin is not expressed in pBluesript, alternate expression vectors involving production of fusion proteins, such as pGEX2T, can be used.

As discussed in this example, approximately 55 % of the toxin gene has been cloned and sequenced; however, using a downstream portion of the known sequence as a probe, the lambda ZAP II library can be rescreened, in order to clone and sequence the remainder of the toxin gene. Construction of a sequence encoding the entire gene can then be performed using methods similar to those discussed above, and the entire recombinant toxin can then be expressed in E. coli.

### EXAMPLE 4

### Detection of H. pylori Toxin Gene by Polymerase Chain Reaction or Probe Hybridization

Diagnostic tests for H. pylori infection can be developed based on primer directed amplification of samples. More specifically, synthetic oligonucleotides selected from the nucleotides set out in sequence Id. no. 1 can be used in a polymerase chain reaction to amplify H. pylori toxin to detectable levels. In this test nondegenerate primer sequences are used for PCR. These nondegenerate primer sequences are selected from the nucleic acids set out in sequence Id. no. 1. More specifically, they may include:

With the use of these primers, a 0.5 kb amplification product is produced, as indicated in Example 3. Alternatively, the toxin gene may be detected by hybridization of clinical samples with the radiolabelled sequence ID Number 1, or a portion thereof.

### EXAMPLE 5

### Use of specific antiserum to the toxin in detection and neutralization of the CB antigen.

Antiserum to the Mr = 87,000 protein subunit was raised in a female White New Zealand rabbit, according to the regimen described previously (Cover, T.L., Dooley, C.P., and Blaser, M.J. (1990) Infect. Immun. 58:603-610). Initially, the rabbit was immunized with Coomasie blue-stained acrylamide gel fragments containing the denatured Mr = 87,000 protein subunit. Subsequently, the rabbit was immunized with the denatured Mr = 87,000 protein subunit that was eluted in distilled water from unstained SDS-PAGE gels, and concentrated by hydrophobic interactive chromatography as described above.

Preimmune and immune serum were assessed by Western blot analysis. Following separation by SDS-PAGE, proteins in H. pylori culture supernatant were transferred to nitrocellulose paper by electroblotting for one hour at one amp. Nitrocellulose paper strips were incubated with sera, washed, incubated with alkaline phosphatase-conjugated anti-human IgG (Boehringer-Mannheim, Indianapolis, IN) or anti-rabbit IgG (Tago, Burlingame, CA), and developed as described by Blake et al. (Blake, M.S., Johnston, K.H., Russel-Jones, G.I., and Gotschlich., E.C. (1984) Anal. Biochem. 136:175-179). Antiserum raised against the purified Mr = 87,000 protein subunit recognized the Mr = 87,000 protein band and no other H. pylori constituents (Figure 3).

Preimmune and immune rabbit sera were also assessed by ELISA. The ELISA was performed with 15 ng purified CB antigen per microtiter well, and the methodology was as previously described (Perez-Perez, G.I., Dworkin, B.M., Chodos, J.E., and Blaser, M.J. (1988) Ann. Intern. Med. 109:465-471 hereby incorporated by reference). Peroxidase-conjugated anti-human IgG (Tago) or anti-rabbit IgG (Boehringer Mannheim) were used as the conjugates. The titer of rabbit serum was defined as the reciprocal of the highest dilution that produced an optical density of greater than 0.2. Using this methodology, the titer of the antiserum was 1:512,000, whereas that of the preimmune serum was <1:200.

Neutralization of vacuolating toxic activity. The toxin preparation used in neutralization assays was prepared by culturing H. pylori 60190 for 48 hours in Brucella broth containing 5% fetal bovine serum, centrifuging the culture, and concentrating the supernatant by ultrafiltration, as previously described (Cover, T.L., Puryear, W., Perez-Perez, G.I., and Blaser, M.J. (1991) Infect. Immun. 59:1264-1270). Sera were heated at 56°C for 30 minutes, diluted in tissue culture medium, and incubated for one hour with an equal volume of concentrated H. pylori supernatant, as previously described (Cover, T.L., Cao, P., Murthy U.K., Sipple, M.S., and Blaser, M.J. (1992) J. Clin. Invest. 90:913-918.) The neutralizing effects of sera on the vacuolating toxic activity were quantitated using the neutral red uptake assay (Cover, T.L., Puryear, W., Perez-Perez, G.I., and Blaser, M.J. (1991) Infect. Immun. 59:1264-1270). Under the assay conditions used, at a 1:64 dilution the antiserum completely neutralized vacuolating toxin activity in supernatant from H. pylori 60190, whereas preimmune serum lacked neutralizing activity (Figure 4). The antiserum also completely neutralized the toxin activity present in culture supernatants from two other toxin-producing H. pylori strains, an indication that the vacuolating toxins produced by various H. pylori isolates are antigenically related.

**Table 4.**

| H. pylori isolates from humans used in the study | | | |
|---|---|---|---|
| Strain | Source | Toxin production | Reciprocal toxin titer^{a} |
| 60190 | ATCC 49503 | + | 320 |
| 85-456 | NTCC 11638 | + | 40 |
| 87-29 | Colorado | + | 160 |
| 87-199 | Colorado | + | 80 |
| 87-81 | Colorado | + | 40 |
| 87-90 | Colorado | + | 40 |
| 86-86 | New York | + | 80 |
| 88-43 | Thailand | + | 20 |
| Tx30a | Texas | - | <10 |
| 87-141 | Colorado | - | <10 |
| 87-75 | Colorado | - | <10 |
| 86-385 | Colorado | - | <10 |
| 86-313 | Colorado | - | <10 |
| 87-6 | Colorado | - | <10 |
| 87-225 | Colorado | - | <10 |
| 87-203 | Colorado | - | <10 |

| | | | |
|---|---|---|---|
| ^{a}The reciprocal toxin titer was defined as the greatest dilution that induced HeLa cell neutral red uptake greater than 3 SD above that induced by medium alone, as described (Cover, T.L., Puryear, W., Perez-Perez, G.I., and Blaser, M.J. (1991) Infect. Immun. 59:1264-1270). | | | |

### Detection of the vacuolating toxin in H. pylori culture supernatants.

The next experiment was designed to determine whether there was a relationship between vacuolating toxin activity and presence of the CB antigen in H. pylori culture supernatants. From a collection of concentrated H. pylori culture supernatants, we selected supernatants from 8 tox⁺ and 8 tox⁻ strains (Cover, T.L., Dooley, C.P., and Blaser, M.J. (1990) Infect. Immun. 58:603-610).

These H. pylori strains (Table 4) were cultured in Brucella broth containing 5% fetal bovine serum, and the culture supernatants were concentrated by ultrafiltration as previously described (Cover, T.L., Dooley, C.P., and Blaser, M.J. (1990) Infect. Immun. 58:603-610).

To quantitate vacuolating toxin activity, dilutions of each of these supernatants were tested using the neutral red assay (Cover, T.L., Puryear, W., Perez-Perez, G.I., and Blaser, M.J. (1991) Infect. Immun. 59:1264-1270). Diluted greater than 1:20, each of the tox⁺ supernatants induced greater than two-fold greater net neutral red uptake by cells than medium alone, whereas each of the tox⁻ supernatants failed to induce significant neutral red uptake when diluted 1:10. To detect the CB antigen, the 16 supernatants were tested by ELISA with a 1:10,000 dilution of antiserum to the Mr = 87,000 protein subunit (Figure 5). The supernatants from tox⁺ strains produced significantly higher optical density values than supernatants from tox⁻ supernatants (0.614 ± 0.105 versus 0.046 ± 0.009, p <0.0001). Western blotting studies confirmed the presence of the Mr = 87,000 band in each of the tox⁺ supernatants, indicating that this is the form of CB antigen under denatured conditions. The lack of overlap between these two groups of supernatants indicates that the CB antigen is the major substituent in H. pylori supernatants that mediates vacuolating toxin activity.

### EXAMPLE 6

### Detection of anti-toxin antibodies in body fluids from H. pylori-infected humans

Previous studies have demonstrated that sera from some, but not all H. pylori-infected persons contain toxin-neutralizing antibodies (Leunk, R.D., Ferguson, M.A., Morgan, D.R., Low, D.E., and Simor, A.E. (1990) J. Clin. Microbiol. 28:1181-1184; Cover, T.L., Cao, P., Murthy U.K., Sipple, M.S., and Blaser, M.J. (1992) J. Clin. Invest. 90:913-918.) We therefore sought to determine the prevalence of antibodies to the purified CB antigen protein in sera from H. pylori-infected and uninfected humans.

Human sera were obtained from forty selected symptomatic patients who had previously undergone gastroduodenal endoscopy at the University Hospital and the Veterans Administration Medical Center, Syracuse, NY. Based on analysis of the gastric biopsy specimens and serologic evaluation of these patients, 20 were infected with H. pylori and twenty were uninfected. The characteristics of these patients and the toxin-neutralizing activities of these sera have been previously described (Cover, T.L., Cao, P., Murthy U.K., Sipple, M.S., and Blaser, M.J. (1992) J. Clin. Invest. 90:913-918.) These 40 sera were tested for IgG reactivity with the purified CB antigen in an ELISA (Figure 6).

The ELISA was performed with 15 ng purified CB antigen per microtiter well, and the methodology was as previously described (Perez-Perez, G.I., Dworkin, B.M., Chodos, J.E., and Blaser, M.J. (1988) Ann. Intern. Med. 109:465-471 hereby incorporated by reference). Peroxidase-conjugated anti-human IgG (Tago) or anti-rabbit IgG (Boehringer Mannheim) were used as the conjugates. The mean recognition of the CB antigen by sera from H. pylori-infected persons was significantly stronger than by sera from uninfected persons (p=0.0009). Sera from approximately half of the H. pylori-infected persons produced optical density values that overlapped those of uninfected persons, whereas sera from other H. pylori-infected persons produced optical density values that did not overlap. This suggests that two populations may be present, and is consistent with the observation that 50%-60% of H. pylori strains are toxigenic in vitro. We then determined whether there was a relationship between recognition of the CB antigen by ELISA and toxin-neutralizing activity, as determined previously in the cell culture assay (Cover, T.L., Cao, P., Murthy U.K., Sipple, M.S., and Blaser, M.J. (1992) J. Clin. Invest. 90:913-918.) For sera from H. pylori-infected persons, ELISA recognition of the CB antigen was significantly associated with toxin-neutralizing activity (p=0.019, r=0.518 by linear regression analysis). In contrast, for sera from uninfected persons, these variables were not significantly associated (p=0.973, r=0.008).

### EXAMPLE 7

### Preparation of an oral vaccine for administration to mammals including humans

We have considered the potential application of the use of the CB protein in the development of a vaccine against H. pylori infections. To limit the effects of gastric acid and proteolytic enzymes on the vaccine preparation, the whole CB protein or a portion thereof can be packaged either in an enteric coated gelatin capsule or administered with sodium bicarbonate (Black et al, "Immunogenicity of Ty21a attenuated Salmonella typhi given with sodium bicarbonate or in enteric-coated capsules." Dev. Biol. Stand. 53:0, 1983). It should be noted that the antigen used in this vaccine could be produced recombinantly. Dosage for adult humans preferably varies from 5.0-50.0 mg of the antigens of the invention.

To enhance delivery of CB protein to the gastrointestinal immune system the protein [or a fragment(s) of the protein] may be incorporated without chemical coupling into biodegradable microspheres that are 5-10 230µm in size that will be ingested orally (Eldridge et al., "Biodegradable microsphere: vaccine delivery systems for oral immunization," Curr. Top. Microbiol. Immunol. 146:59, 1989). The microspheres are composed of co-polymers of glycolic and lactic acids which are degraded into original components by hydrolysis. Adjusting the ratio of glycolic to lactic acids within the co-polymers varies the rate of hydrolysis from several hours to several months. Thus, both fast- and slow-releasing microspheres can be created. The use of a mixture of both fast- and slow-releasing microspheres will then be used to allow for induction of both a primary and secondary immune response with a single oral immunization.

### EXAMPLE 8

### Preparation of a parenteral vaccine for administration to mammals including humans

Although for gastrointestinal pathogens, orally administered vaccines appear to be preferable, for several other infectious agents, parenteral vaccine show efficacy. A component of the bacterium Salmonella typhi, the cause of typhoid fever, has been purified and used as a parenteral-administered vaccine. This component, the Vi capsular polysaccharide, is highly efficacious (Klugman KP, et al., "Protective activity of Vi capsular polysaccharide vaccine against typhoid fever," Lancet 1987;2:165-69"). The Salk vaccine for polio is administered parenterally and it prevents the disease of polio, although having little or no effect on becoming infected with the polioviruses. Parenteral vaccines also have efficacy, although limited, in preventing cholera.

For H. pylori, a parenteral vaccine could include CB protein or fragments thereof. A toxoid preparation could also be prepared, analogous to the use of diphtheria or tetanus toxoids. The protein(s) or fragment(s) could be administered with an adjuvant or by itself in a suitable buffer. Adjuvants include, but are not limited to, muramyl dipeptide, concanavalin A, DEAE dextran, lipid polyvalent cations, or hydrocarbons such as hexadecane.

H. pylori vaccine could be given to humans as 1.0 mg (range 0.5-5.0 mg) of antigen (CB protein) in 1 ml of phosphate buffered saline (pH 7.4). With a suitable antigen, only a single dose may be needed, but multiple doses with or without adjuvants could be considered.

### EXAMPLE 9

### Test kits for detection of antibodies to H. pylori toxin, and for detection of H. pylori toxin

Specific test kits are constructed for detecting antibodies using several different techniques for detection. One test kit for antibody detection is comprised of a compartmented enclosure containing a plurality of wells, plates which were coated prior to use with CB protein or an antigenic fragment thereof, and ELISA materials for enzyme detection consisting of peroxidase-labeled goat anti-human IgG and a color change indicator consisting of ABTS in McIlvain's buffer with 0.005 percent hydrogen peroxide. It should be noted that the antigen used in an assay could made recombinantly. Naturally, other enzymes and developers could have been used. For instance, alkaline phosphatase-labeled goat anti-human IgG could be used in conjunction with p-nitrophenyl phosphate in diethanolamine and magnesium chloride buffer.

A second test kit for detecting antibodies using the Western blot technique is comprised of a container, cover, nitrocellulose sheet, and a polyacrylamide slab gel in the presence of sodium dodecyl sulfate, surfactants, pH modifiers, dried nonfat milk and materials for enzyme detection including a color change indicator consisting of DAB in Tris with hydrogen peroxide. This Western blot analysis kit also contains peroxidase-labeled goat or rabbit anti-human immunoglobulin and a source of CB protein or antigenic fragment thereof.

Another H. pylori specific test kit for detecting antibodies using the indirect immunofluorescence assay may include a compartmental container with CB protein or antigenic fragments thereof as antigens, human test serum, phosphate buffered saline and fluorescein-conjugated goat anti-human IgG.

Finally, a different H. pylori specific test kit for detecting antibodies uses liposomes and comprises a container, human test serum, fluorescent marker- (or enzyme- or substrate-) filled liposomes with antigens on their surface, and a surface-active agent. In this assay the container might be a precoated tube or well with goat anti-human IgG.

H. pylori specific test kits are constructed for detecting H. pylori toxin using several different techniques for detection. One test kit for detection of H. pylori toxin comprises a compartmented enclosure containing a plurality of wells, plates that could be coated with the sample to be tested, a hyperimmune antiserum (or monoclonal antibodies) to CB protein or antigenic fragment thereof, anti-rabbit immunoglobulin and appropriate ELISA materials such as those discussed above in this example.

A second test kit for detecting H. pylori toxin using the Western blot technique is comprised of a container, cover, nitrocellulose sheet, and a polyacrylamide slab gel in the presence of sodium dodecyl sulfate, surfactants, pH modifiers, dried nonfat milk and materials for enzyme detection including a color change indicator consisting of DAB in Tris with hydrogen peroxide. This Western blot analysis kit also contains goat anti-rabbit immunoglobulin and a source of hyperimmune antiserum to CB protein or antigenic fragment thereof.

Another H. pylori specific test kit for detecting the toxin using the latex agglutination assay may include a compartmental container, hyperimmune serum to CB protein or antigenic fragment thereof conjugated to latex beads, and phosphate buffered saline or water.

### EXAMPLE 10

### Inhibition of H. pylori vacuolating toxin activity by bafilomycin A1.

The cellular vacuoles that form in response to H. pylori vacuolating toxin are acidic in pH. (Cover TL, Halter SA, MJ Blaser. 1992. "Characterization of HeLa cell vacuoles induced by H. pylori broth culture supernatant." Human Pathology 23:1004-1010). The maintenance of pH gradients within compartments of eukaryotic cells typically is dependent upon the activity of a vacuolar-type proton-transporting ATPase (Mellman I, Fuchs R, and A Helenius. 1986. "Acidification of the endocytic and exocytic pathways." Annu. Rev. Biochem. 55:663-700). We hypothesized that the vacuolar ATPase of eukaryotic cells might be important in the formation and maintenance of H. pylori toxin-induced vacuoles. Therefore, we tested the effects of vacuolar ATPase inhibitors upon H. pylori toxin-induced cell vacuolation.

HeLa cells were incubated with H. pylori toxin in the presence of nine different inhibitors of ion-transporting ATPases (bafilomycin A1, N-ethylmaleimide (NEM), 7-chlor-4-nitrobenz2-oxa-1,3-diazole (NBD chloride), N,N'dicyclohexylcarbodiimide pentachlorophenol complex (DCCD), sodium nitrate, ouabain,digoxin, sodium orthovanadate, omeprazole, and oligomycin).

More specifically, H. pylori 60190, a well characterized strain that produces the vacuolating toxin, was cultured for 48 hours at 37°C in Brucella broth supplemented with 5% fetal bovine serum in a 5% CO₂ atmosphere. After centrifugation of the culture, supernatant was concentrated 30-fold by ultrafiltration and passed through a 0.2 micron filter. Supernatants were stored at -70.0°C prior to testing in tissue culture assays. Purified toxin was prepared from H. pylori 60190 as previoulsly described except that gel filtration chromatography was performed with a SUPEROSE 6 HR 10/50 column (Pharmacia) instead of SUPEROSE 12HR 10/50 column (Pharmacia).

HeLa cells were cultured in Eagle's modified minimal essential medium with Earle's salts containing 10% fetal bovine serum and 25 mM HEPES buffer (pH 7.2) in a 5% CO₂ atmosphere. In experiments involving purified H. pylori toxin, the medium was supplemented with 10mM ammonium chloride to potentiate activity. After preincubation of the cells with ATPase inhibitors for one hour, concentrated culture supernatant or purified toxin from H. pylori 60190 was added and cells were incubated for an additional eighteen hours at 37°C. Vaculation was assessed visually by inverted light microscopy (200x magnification), or quantitated using a neutral red uptake assay. (Cover et al., Infect. Immun, 59:1264-1270 (1991)). In a microscopic assay, inhibition of H. pylori vacuolating toxin activity was defined by the stringent criterion of visible vacuoles in less the ten percent of the cells.

Inhibitors of predominantly vacuolar-type ATPases inhibited the formation of vacuoles in response to the H. pylori toxin, and reversed the vacuolation induced by the toxin. Of the vacuolar ATPase inhibitors tested, bafilomycin A1 was the most potent inhibitor of toxin-induced vacuolation (minimum inhibitory concentration = 25 nM). Vacuolating toxin activity was inhibited by higher concentrations of other vacuolar ATPase inhibitors, including
N-ethylmaleimide,
7-chloro-4-nitrobenz-2-oxa-1,3-diazole,
N,N'-dicyclohexylcarbodiimide, and sodium nitrate. In contrast, F₁F₀-type or P-type ATPase inhibitors did not inhibit toxin activity. (See table 5.)

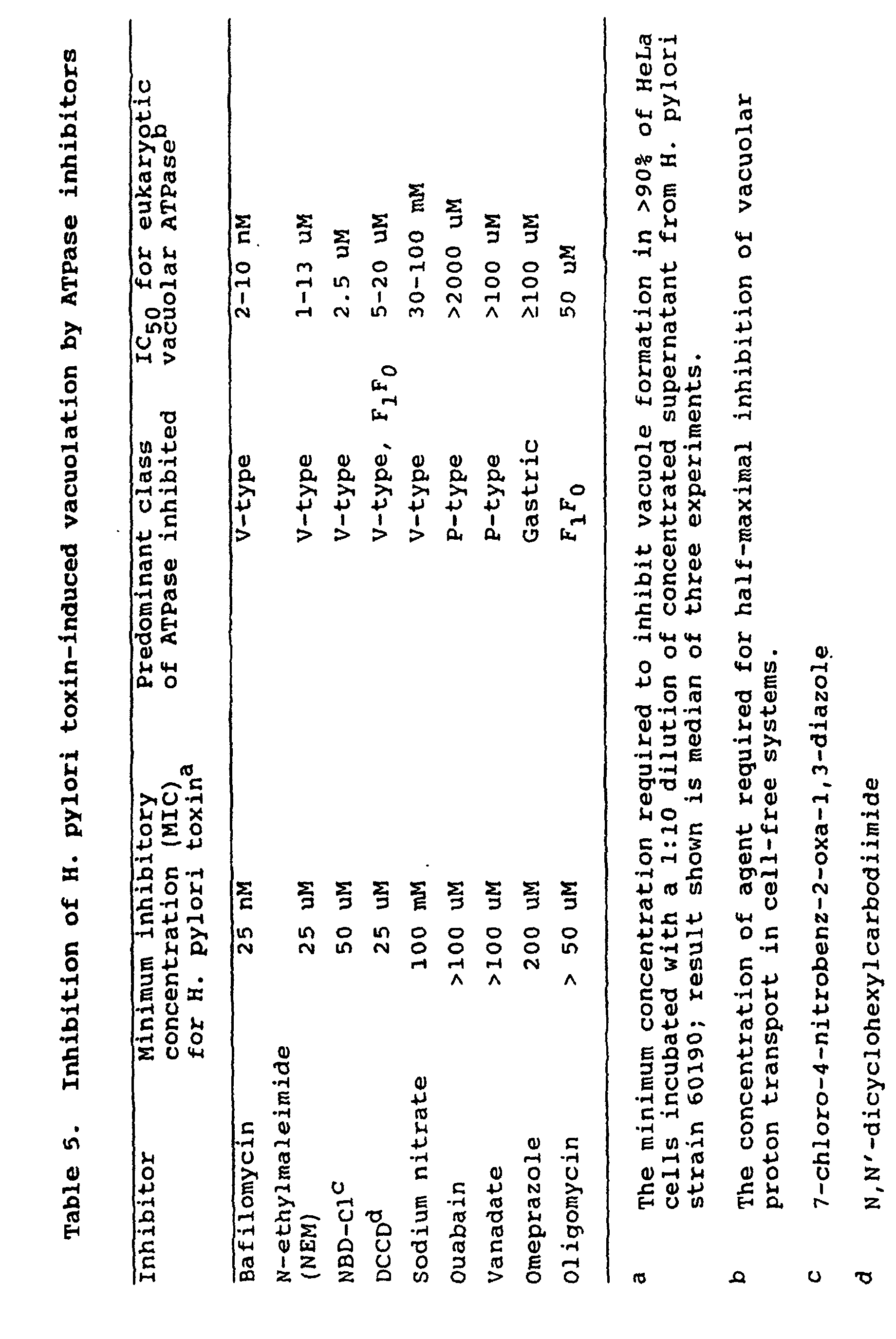

In summary, inhibitors of vacuolar-type ATPase, exemplified by bafilomycin Al, are inhibitors of cellular damage induced by H.pylori vacuolating toxin, and may be useful therapeutic agents in the treatment of H.pylori-associated gastroduodenal disease.

Although the invention has been described primarily in connection with special and preferred embodiments, it will be understood that it is capable of modification without departing from the scope of the invention. The following claims are intended to cover all variations, uses, or adaptations of the invention, following, in general, the principles thereof and including such departures from the present disclosure as come within known or customary practice in the field to which the invention pertains, or as are obvious to persons skilled in the field.

## Claims

1. A purified antigen that specifically induces vacuolation of eukaryotic cells wherein said antigen is **characterized by** a molecular weight of greater than 972,000 (as determined by gel filtration chromatography under nondenaturing conditions), and wherein said antigen when denatured is **characterized by** a molecular weight of about 87,000 (as determined by sodium dodecyl sulfate polyacrylamide gel electrophoresis under reducing conditions) and wherein said antigen has an isolectric point of approximately 6.1, and fragments and analogs thereof capable of specifically inducing vacuolation of eukaryotic cells.

2. The purified antigen of claim 1 wherein said antigen includes the amino terminal sequence shown in Sequence Id. No. 2.

3. The purified antigen of claim 1 or 2, wherein said antigen includes the amino terminal sequence shown in Sequence Id. No. 2 and internal amino acid sequence selected from the group consisting of the amino acid sequences shown in Sequence Id. No. 16 or 17.

4. The purified antigen of claim 1 that specifically induces vacuolation of eukaryotic cells wherein said antigen is greater than 5000 times more purified than in broth culture supernatant.

5. The purified antigen of claim 1 wherein said antigen includes an amino acid sequence that is encoded by a nucleotide sequence as set out in Sequence Id No. 1.

6. An isolated nucleic acid encoding the purified antigen of claim 1, or a fragment thereof.

7. An antibody that specifically binds to *H. pylori* toxin made by the process comprising: producing antiserum to the purified antigen of claim 1 and isolating said antibody from said antiserum.

8. An antibody that specifically binds to *H. pylori* toxin made by the process comprising: producing monoclonal antibodies to the purified antigen of claim 1 and isolating said antibody.

9. A vaccine against *H*. *pylori* infection, said vaccine comprising an amount of the purified antigen of claim 1 with a pharmaceutically acceptable carrier effective to induce production of protective antibodies against *H*. *pylori* infection.

10. A diagnostic test kit for detecting *H. pylori* infection, said test kit comprising:
a) an immobilized purified antigen of claim 1;
b) means for passing bodily fluids taken from a human or animal to be tested over said immobilized antigen; and
c) means for detecting immunoglobulin from said fluid bound to said immobilized antigen.

11. A diagnostic test kit for detecting *H*. *pylori* toxin in a sample from a human or animal, said test kit comprising:
a) the antibody of claim 7 that specifically binds to a toxin that induces cell vacuolation;
b) means for contacting said sample with said antibody; and
c) means for detecting toxin from said fluid bound to said antibody.

12. The kit of claim 11 where sample is bodily fluids, tissue, *H. pylori* culture supernatant or other *H. pylori* preparations.

13. A diagnostic test kit for detecting *H*. *pylori* toxin-encoding nucleic acids in sample, said test kit comprising:
a) two single stranded PCR primer oligonucleotides being part of the nucleic acid sequence set out in Sequence Id. No. 1.
b) means for conducting PCR reaction; and
c) means for detecting *H. pylori* toxin-encoding nucleic acids.

14. Synthetic oligonucleotides represented by DNA sequences set out as sequence identification numbers 21 and 22 useful in detecting *H*. *pylori* vacuolating toxin-encoding nucleic acids.

15. A method of detecting *H. pylori* vacuolating toxin nucleic acids in a sample from a human subject by primer directed amplification wherein said sample is amplified with dual primers consisting of two single stranded PCR primer oligonucleotides being part of the isolated nucleic acid of claim 6, and following said amplification hybridizing a single stranded oligonucleotide probe to said amplification product and detecting said hybridized probe.

16. A method to detect anti-toxin antibodies in a sample of bodily fluid taken from a human or animal comprising:
a) contacting purified antigen of claim 1 bound to a solid phase with said sample;
b) incubating said contactants of Step (A) to bind said antibodies to said bound purified antigen; and
c) detecting said bound anti-toxin antibodies

17. A method to detect a toxin that induces vacuolation of eukaryotic cells in a bound sample comprising:
a) contacting the antibody of claim 7 with said bound sample;
b) incubating said contactants of Step (A) to form antigen-antibody complexes;
c) detecting said antigen-antibody complexes.

18. The method of claim 15, wherein said sample is selected from the group consisting of bodily fluids, *H. pylori* culture supernatant, or other *H. pylori* preparations.

19. A method of detecting infection caused by a toxin-producing *H. pylori* strain in a patient comprising:
a) contacting the purified antigen of claim 1 bound to a solid phase with a sample of bodily fluid from said patient;
b) incubating said contactants of Step (A) to bind said antibodies to said bound antigen;
c) detecting said bound anti-toxin antibodies;
d) comparing the amount of detected anti-toxin antibody with a standard to detect infection caused by a toxin-producing *H*. *pylori* strain.

20. A method to detect anti-toxin antibodies in a patient and thereby to determine the susceptibility of a patient to develop peptic ulcer disease, gastric carcinoma or other clinical consequences of *H*. *pylori* infection comprising.
a) contacting the purified antigen of claim 1 bound to a solid phase with a sample of bodily fluid from said parent;
b) incubating said contactants of Step (A) to bind said antibodies to said bound antigen;
c) detecting said bound anti-toxin antibodies;
d) comparing the amount of detected anti-toxin antibody with a standard to determine the susceptibility of a patient to develop peptic ulcer disease, gastric carcinoma or other clinical consequence of *H*. *pylori* infection.

21. Use of the antigen according to any of claims 1 to 5 for preparing a vaccine for immunizing animals, including humans, against *H. pylori*.

22. Use according to claim 21, wherein said vaccine is administered enterally.

23. Use according to claim 21, wherein said vaccine is administered parenterally

24. Use of an antibody according to any of claims 7 or 8 for preparing a medicament for neutralizing a toxin that induces vacuolation of eukaryotic cells.

25. A method for recombinant production of the antigen of claim 1 or fragments thereof, by expressing all or part of the DNA sequence set out in Sequence Id No. 1 in a suitable expression system.

## Patentansprüche

1. Ein gereinigtes Antigen, das spezifisch die Vakuolisierung eukaryotischer Zellen induziert, wobei das Antigen durch ein Molekulargewicht, das größer als 972 000 ist (mittels Gelfiltrationschromatographie unter nicht-denaturierenden Bedingungen bestimmt), charakterisiert ist, und wobei das Antigen in denaturierter Form durch ein Molekulargewicht von ungefähr 87 000 (mittels Natriumdodecylsulfat-Polyacrylamid-Gelelektrophorese unter reduzierenden Bedingungen bestimmt) charakterisiert ist, und wobei das Antigen einen isoelektrischen Punkt von ungefähr 6,1 aufweist und Fragmente und Analoga davon, die fähig sind, spezifisch die Vakuolisierung eukaryotischer Zellen zu induzieren.

2. Das gereinigte Antigen gemäß Anspruch 1, wobei das Antigen die aminoterminale Sequenz, die in Sequenz ID Nr. 2 gezeigt ist, einschließt.

3. Das gereinigte Antigen gemäß Anspruch 1 oder 2, wobei das Antigen die aminoterminale Sequenz, die in Sequenz ID Nr. 2 gezeigt ist, und eine innere Aminosäuresequenz, ausgewählt aus der Gruppe der Aminosäuresequenzen, die in Sequenz ID Nr. 16 oder 17 gezeigt sind, einschließt.

4. Das gereinigte Antigen gemäß Anspruch 1, das spezifisch die Vakuolisierung eukaryotischer Zellen induziert, wobei das Antigen mehr als 5 000-fach reiner als in Kulturbrühe-Überstand vorliegt.

5. Das gereinigte Antigen gemäß Anspruch 1, wobei das Antigen eine Aminosäuresequenz einschließt, die durch eine Nukleotidsequenz nach Sequenz ID NO. 1 kodiert ist.

6. Eine isolierte Nukleinsäure, die für das gereinigte Antigen gemäß Anspruch 1 oder ein Fragment davon kodiert.

7. Ein Antikörper, der spezifisch an *H*. *pylori*-Toxin bindet und durch ein Verfahren hergestellt ist, das das Herstellen von Antiserum gegen das gereinigte Antigen gemäß Anspruch 1 und Isolieren des Antikörpers aus dem Antiserum umfasst.

8. Ein Antikörper, der spezifisch an *H*. *pylori*-Toxin bindet und durch ein Verfahren hergestellt ist, das das Herstellen von monoklonalen Antikörpern gegen das gereinigte Antigen gemäß Anspruch 1 und Isolieren des Antikörpers umfasst.

9. Ein Impfstoff gegen eine *H*. *pylori*-Infektian, wobei der Impfstoff eine Menge des gereinigten Antigens gemäß Anspruch 1 mit einem pharmazeutisch akzeptablen Träger umfasst, die wirksam die Produktion von protektiven Antikörpern gegen eine *H*. *pylori*-Infektion induziert.

10. Ein diagnostischer Test-Kit zum Nachweis von einer *H*. *pylori*-Infektion, wobei der Test-Kit umfasst:
a) ein immobilisiertes gereinigtes Antigen gemäß Anspruch 1;
b) Mittel, die erlauben, dass Körperflüssigkeiten, die von einem Menschen oder Tier stammen und untersucht werden sollen, das immobilisierte Antigen passieren können; und
c) Mittel, die einen Nachweis von an das immobilisierte Antigen gebundenem Immunoglobulin aus der Flüssigkeit erlauben.

11. Ein diagnostischer Test-Kit zum Nachweis von *H*. *pylori*-Toxin in einer menschlichen oder tierischen Probe, wobei der Testkit umfasst:
a) den Antikörper gemäß Anspruch 7, der spezifisch an ein Toxin bindet, das die Vakuolisierung von Zellen induziert;
b) Mittel, die einen Kontakt der Probe mit dem Antikörper erlauben; und
c) Mittel, die einen Nachweis des an den Antikörper gebundenen Toxins aus der Flüssigkeit erlauben.

12. Der Kit gemäß Anspruch 11, wobei die Probe Körperflüssigkeiten, Gewebe, *H*. *pylori*-Kulturüberstand oder andere *H*. *pylori*-Präparationen sind.

13. Ein diagnostischer Test-Kit zum Nachweis von für *H*. *pylori*-Toxin kodierenden Nukleinsäuren in einer Probe, wobei der Test-Kit umfasst:
a) zwei einzelsträngige PCR-Primer-Oligonukleotide, die Teil der Nukleinsäuresequenz gemäß der Sequenz ID NO. 1 sind;
b) Mittel, die es erlauben, die PCR-Reaktion durchzuführen; und
c) Mittel, die den Nachweis von für *H*. *pylori*-Toxin kodierenden Nukleinsäuren erlauben.

14. Synthetische Oligonukleotide, die durch die DNA-Sequenzen nach den Sequenz ID Nummern 21 und 22 repräsentiert sind und für den Nachweis von Nukleinsäuren aus *H*. *pylori*, die für vakuolisierendes Toxin kodieren, nützlich sind.

15. Ein Verfahren zum Nachweis von Nukleinsäuren aus *H. pylori*, die für vakuolisierendes Toxin kodieren, in einer vom Menschen stammenden Probe durch Primer-vermittelte Amplifikation, wobei die Probe mit zwei Primem amplifiziert wird, die aus zwei einzelsträngigen PCR-Primer-Oligonukleotiden bestehen und Teil der isolierten Nukleinsäure gemäß Anspruch 6 sind, und Hybridisieren einer einzelsträngigen Oligonukleotidsonde mit dem Amplfikationsprodukt auf die Amplifikation folgend und Nachweisen der hybridisierten Sonde.

16. Ein Verfahren zum Nachweis von Anti-Toxin-Antikörpem in einer Körperflüssigkeitprobe, die aus einem Menschen oder Tier stammt, umfassend:
a) Kontaktieren des gereinigten Antigens gemäß Anspruch 1, das an eine Festphase gebunden ist, mit der Probe;
b) Inkubieren der miteinander in Kontakt stehenden Agenzien aus Schritt a), um die Antikörper an das gereinigte Antigen zu binden; und
c) Nachweisen der gebundenen Anti-Toxin-Antikörper.

17. Ein Verfahren zum Nachweis eines Toxins in einer gebundenen Probe, das die Vakuolisierung eukaryotischer Zellen induziert, umfassend:
a) Kontaktieren des Antikörpers gemäß Anspruch 7 mit der gebundenen Probe;
b) Inkubieren der miteinander in Kontakt stehenden Agenzien aus Schritt a), um Antigen-Antikörper-Komplexe zu bilden;
c) Nachweisen der Antigen-Antikörper-Komplexe.

18. Das Verfahren gemäß Anspruch 15, wobei die Probe aus einer Gruppe ausgewählt wird, die aus Körperflüssigkeiten, *H*. *pylori*-Kulturüberstand oder anderen *H*. *pylori*-Präparationen besteht.

19. Ein Verfahren zum Nachweis einer Infektion, die durch ein Toxin-bildenden *H*. *pylori*-Stamm in einem Patienten verursacht ist, umfassend:
a) Kontaktieren des gereinigten Antigens aus Anspruch 1, das an eine Festphase gebunden ist, mit einer Körperflüssigkeitprobe aus dem Patienten;
b) Inkubieren der miteinander in Kontakt stehenden Agenzien aus Schritt a), um die Antikörper an das gebundene Antigen zu binden;
c) Nachweisen der gebundenen Anti-Toxin-Antikörper;
d) Vergleichen der Menge an nachgewiesenem Anti-Toxin-Antikörper mit einem Standard, um eine Infektion, die durch einen Toxin bildenden *H*. *pylori*-Stamm verursacht ist, nachzuweisen.

20. Ein Verfahren zum Nachweis von Anti-Toxin-Antikörpem in einem Patienten zur Bestimmung der Empfänglichkeit eines Patienten, Magengeschwüre, Magenkarzinome oder andere klinische Konsequenzen aus eine *H*. *pylori*-Infektion zu entwickeln, umfassend:
a) Kontaktieren des gereinigten Antigens gemäß Anspruch 1, das an eine Festphase gebunden ist, mit einer Körperflüssigkeitprobe aus dem Patienten;
b) Inkubieren der miteinander in Kontakt stehenden Agenzien aus Schritt a), um die Antikörper an das Antigen zu binden;
c) Nachweisen der gebundenen Anti-Toxin-Antikörper;
d) Vergleichen der Menge an nachgewiesenem Anti-Toxin-Antikörper mit einem Standard zur Bestimmung der Empfänglichkeit eines Patienten, Magengeschwüre, Magenkarzinome oder andere klinische Konsequenzen aus eine *H*. *pylori*-Infektion zu entwickeln.

21. Verwendung des Antigens gemäß irgendeinem der Ansprüche 1 bis 5 zur Herstellung eines Impfstoffs zur Immunisierung von Tieren, einschließlich Menschen, gegen *H. pylori*.

22. Verwendung gemäß Anspruch 21, wobei der Impfstoff enteral verabreicht wird.

23. Verwendung gemäß Anspruch 21, wobei der Impfstoff parenteral verabreicht wird.

24. Verwendung eines Antikörpers gemäß irgendeinem der Ansprüche 7 oder 8 zur Herstellung eines Medikaments zur Neutralisierung eines Toxins, das die Vakuolisierung von eukaryotischen Zellen induziert.

25. Ein Verfahren zur rekombinanten Herstellung des Antigens gemäß Anspruch 1 oder Fragmenten davon, durch Expression der gesamten oder von Teilen der DNA nach Sequenz ID NO. 1 in einem geeigneten Expressionssystem.

## Revendications

1. Antigène purifié qui provoque spécifiquement la vacuolisation de cellules eucaryotes, où ledit antigène est **caractérisé par** un poids moléculaire supérieur à 972 000 (tel que déterminé par chromatographie de filtration sur gel dans des conditions non dénaturantes) et où ledit antigène, lorsqu'il est dénaturé, est **caractérisé par** un poids moléculaire d'environ 87 000 (tel que déterminé par électrophorèse sur gel de polyacrylamide-dodécylsulfate de sodium dans des conditions réductrices) et où ledit antigène a un point isoélectrique d'approximativement 6,1 et des fragments et des analogues de celui-ci capables de provoquer spécifiquement la vacuolisation de cellules eucaryotes.

2. Antigène purifié selon la revendication 1, où ledit antigène comprend la séquence amino terminale illustrée par la Séquence Id. No. 2.

3. Antigène purifié selon la revendication 1 ou 2, où ledit antigène comprend la séquence amino terminale illustrée par la Séquence Id. No. 2 et une séquence interne d'acides aminés choisie dans le groupe constitué des séquences d'acides aminés illustrées par la Séquence Id. No. 16 ou 17.

4. Antigène purifié selon la revendication 1 qui provoque spécifiquement la vacuolisation de cellules eucaryotes, où ledit antigène est plus de 5000 fois plus purifié que dans le surnageant de culture en bouillon.

5. Antigène purifié selon la revendication 1, où ledit antigène comprend une séquence d'acides aminés qui est codée par une séquence de nucléotides telle qu'illustrée par la Séquence Id. No. 1.

6. Acide nucléique isolé codant pour l'antigène purifié selon la revendication 1, ou pour un fragment de celui-ci.

7. Anticorps qui se lie spécifiquement à la toxine de *H*. *pylori*, fabriqué par le procédé comprenant : la production d'antisérum dirigé contre l'antigène purifié selon la revendication 1 et l'isolement dudit anticorps à partir dudit antisérum.

8. Anticorps qui se lie spécifiquement à la toxine de *H*. *pylori*, fabriqué par le procédé comprenant : la production d'anticorps monoclonaux dirigés contre l'antigène purifié selon la revendication 1 et l'isolement dudit anticorps.

9. Vaccin contre une infection à *H*. *pylori*, ledit vaccin comprenant une quantité de l'antigène purifié selon la revendication 1 avec un vecteur pharmaceutiquement acceptable efficace pour provoquer la production d'anticorps protecteurs contre une infection à *H. pylori*.

10. Kit de test de diagnostic destiné à la détection d'une infection à *H. pylori*, ledit kit de test comprenant :
a) un antigène purifié immobilisé selon la revendication 1 ;
b) un moyen de passer les liquides corporels prélevés chez un homme ou chez un animal pour être testés sur ledit antigène immobilisé ; et
c) un moyen de détecter une immunoglobuline provenant dudit liquide lié au dit antigène immobilisé.

11. Kit de test de diagnostic destiné à la détection de la toxine de *H. pylori* dans un échantillon provenant d'un homme ou d'un animal, ledit kit de test comprenant :
a) l'anticorps selon la revendication 7 qui se lie spécifiquement à une toxine qui provoque la vacuolisation cellulaire ;
b) un moyen de mettre en contact ledit échantillon avec ledit anticorps ; et
c) un moyen de détecter la toxine provenant dudit liquide lié au dit anticorps.

12. Kit selon la revendication 11, où l'échantillon est des liquides corporels, du tissu, du surnageant de culture de *H. pylori* ou d'autres préparations de *H. pylori*.

13. Kit de test de diagnostic destiné à la détection d'acides nucléiques codant pour la toxine de *H. pylori* dans un échantillon, ledit kit de test comprenant :
a) deux oligonucléotides d'amorces de PCR simple brin faisant partie de la séquence d'acide nucléique illustrée par la Séquence Id. No. 1.
b) un moyen de conduire une réaction de PCR ; et
c) un moyen de détecter des acides nucléiques codant pour la toxine de *H. pylori*.

14. Oligonucléotides synthétiques représentés par les séquences d'ADN illustrées par les numéros d'identification de séquence 21 et 22, utiles pour la détection d'acides nucléiques codant pour la toxine vacuolisante de *H. pylori*.

15. Méthode de détection d'acides nucléiques de la toxine vacuolisante de *H. pylori* dans un échantillon provenant d'un sujet humain par une amplification dirigée par amorce, dans laquelle ledit échantillon est amplifié avec des amorces doubles constituées de deux oligonucléotides d'amorces de PCR simple brin faisant partie de l'acide nucléique isolé selon la revendication 6 et suivant ladite amplification hybridant une sonde oligonucléotidique simple brin au dit produit d'amplification et la détection de ladite sonde hybridée.

16. Méthode pour détecter des anticorps anti-toxine dans un échantillon de liquide corporel prélevé chez un homme ou chez un animal comprenant :
a) la mise en contact de l'antigène purifié selon la revendication 1 lié à une phase solide avec ledit échantillon ;
b) l'incubation desdits contactants de l'Étape (A) pour lier lesdits anticorps au dit antigène purifié lié ; et
c) la détection desdits anticorps anti-toxine liés.

17. Méthode pour détecter une toxine qui provoque la vacuolisation de cellules eucaryotes dans un échantillon lié comprenant :
a) la mise en contact de l'anticorps selon la revendication 7 au dit échantillon lié ;
b) l'incubation desdits contactants de l'Étape (A) pour former des complexes antigène-anticorps ;
c) la détection desdits complexes antigène-anticorps.

18. Méthode selon la revendication 15, dans laquelle ledit échantillon est choisi dans le groupe constitué de liquides corporels, de surnageant de culture de *H*. *pylori* ou d'autres préparations de *H*. *pylori*.

19. Méthode de détection d'une infection provoquée par une souche de *H. pylori* produisant une toxine chez un patient, comprenant :
a) la mise en contact de l'antigène purifié selon la revendication 1 lié à une phase solide avec un échantillon de liquide corporel provenant dudit patient ;
b) l'incubation desdits contactants de l'Étape (A) pour lier lesdits anticorps au dit antigène lié ;
c) la détection desdits anticorps anti-toxine liés ;
d) la comparaison de la quantité d'anticorps anti-toxine détecté avec un étalon pour détecter une infection provoquée par une souche de *H*. *pylori* produisant une toxine.

20. Méthode pour détecter des anticorps anti-toxine chez un patient et de cette manière pour déterminer la susceptibilité d'un patient de développer une pathologie d'ulcère gastro-duodénal, un cancer gastrique ou d'autres conséquences cliniques d'une infection à *H. pylori*, comprenant :
a) la mise en contact de l'antigène purifié selon la revendication 1 lié à une phase solide avec un échantillon de liquide corporel provenant dudit patient ;
b) l'incubation desdits contactants de l'Étape (A) pour lier lesdits anticorps au dit antigène lié ;
c) la détection desdits anticorps anti-toxine liés ;
d) la comparaison de la quantité d'anticorps anti-toxine détecté avec un étalon pour déterminer la susceptibilité d'un patient de développer une pathologie d'ulcère gastro-duodénal, un cancer gastrique ou d'autres conséquences cliniques d'une infection à *H. pylori*.

21. Utilisation de l'antigène selon l'une quelconque des revendications 1 à 5 pour préparer un vaccin destiné à immuniser des animaux, y compris des hommes, contre *H. pylori*.

22. Utilisation selon la revendication 21, dans laquelle ledit vaccin est administré par voie entérale.

23. Utilisation selon la revendication 21, dans laquelle ledit vaccin est administré par voie parentérale.

24. Utilisation d'un anticorps selon l'une quelconque des revendications 7 ou 8 pour préparer un médicament destiné à neutraliser une toxine qui provoque la vacuolisation de cellules eucaryotes.

25. Méthode de production recombinante de l'antigène selon la revendication 1 ou de fragments de celui-ci, en exprimant la totalité ou une partie de la séquence d'ADN illustrée par la Séquence Id. No. 1 dans un système d'expression approprié.
